(19) Europäisches Patentamt
European Patent Office
Office européen des brevets

(11) Veröffentlichungsnummer: **0 229 947 B1**

(12) # EUROPÄISCHE PATENTSCHRIFT

(45) Veröffentlichungstag der Patentschrift: **13.03.91**

(21) Anmeldenummer: **86116703.9**

(22) Anmeldetag: **02.12.86**

Die Akte enthält technische Angaben, die nach dem Eingang der Anmeldung eingereicht wurden und die nicht in dieser Patentschrift enthalten sind.

(51) Int. Cl.5: **C07C 275/24**, C07C 217/14, C07C 233/00, C07C 255/03, C07D 209/08, C07D 295/08, C07D 307/12, C07D 285/06, A61K 31/00

(54) **Neue kristalline Salze von Aryloxy-propanolaminen, Verfahren zu ihrer Herstellung und ihre Verwendung.**

(30) Priorität: **13.12.85 DE 3544172**

(43) Veröffentlichungstag der Anmeldung: **29.07.87 Patentblatt 87/31**

(45) Bekanntmachung des Hinweises auf die Patenterteilung: **13.03.91 Patentblatt 91/11**

(84) Benannte Vertragsstaaten: **AT BE CH DE ES FR GB GR IT LI LU NL SE**

(56) Entgegenhaltungen:
**EP-A- 0 007 294**
**AT-B- 350 539**
**DE-A- 2 322 053**
**US-A- 2 517 856**
**EP-A- 0 007 605**
**DD-A- 118 614**
**DE-B- 2 458 624**

(73) Patentinhaber: **RORER INTERNATIONAL (OVERSEAS) INC.**
**1209 Orange Street**
**Wilmington Delaware 19899(US)**

(72) Erfinder: **Zölss, Gerhard, Dr.**
**Ziegeleistrasse 72/2**
**A-4020 Linz(AT)**
Erfinder: **Pfarrhofer, Gerhard**
**Schumpeterstrasse 15**
**A-4040 Linz(AT)**

(74) Vertreter: **Müllner, Erwin, Dr. et al**
**Patentanwälte Dr. Erwin Müllner Dipl.-Ing. Werner Katschinka Postfach 159 Weihburggasse 9**
**A-1010 Wien(AT)**

**EP 0 229 947 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft neue kristalline Salze von Aryloxy-propanolaminen mit Diphenylessigsäure, ein Verfahren zu deren Herstellung und die Verwendung dieser Salze zur Herstellung von chemisch reinen Aryloxy-propanolaminen oder deren pharmazeutisch verträglichen Salzen.

Eine Anzahl von verschiedenen substituierten 1-Aryloxy-3-amino-2-propanolen und deren pharmazeutisch verträglichen Salze weisen interessante pharmakologische Eigenschaften auf und haben bereits Aufnahme in den Arzneimittelschatz gefunden. Verbindungen aus dieser Substanzklasse stehen für verschiedene Indikationsgebiete zur Verfügung, wobei insbesondere die Beta-Rezeptoren-blockierende Wirkung im Vordergrund steht. Beispiele für hochwirksame Verbindungen sind N′-(3-Acetyl-4-(3-((1,1-dimethylethyl)amino)-2-hydroxypropoxy)phenyl)-N,N-diethylharnstoff (Celiprolol), 1-(4-(2-Methoxyethyl)phenopxy-3-(-(1-methylethyl)-amino)-2-propanol (Metoprolol) oder 1-((1-Methylethyl)amino)-3-(1-naphtyloxy)-2-propanol (Propanolol).

Für die Herstellung von Aryloxy-propanolaminen stehen verschiedene Syntheseverfahren zur Verfügung, die am Beispiel des Celiprolol u. a. in der DE-PS-2,458.624, AT-PS 335.465 oder AT-PS 335.464 beschrieben sind.

An die Reinheit der Aryloxy-prolanolaminen werden in Hinblick auf ihre medizinische Verwendung sehr hohe Anforderungen gestellt. Insbesondere dürfen Nebenprodukte aus der Synthese in den Präparaten, die in der Humanmedizin zur Anwendung gelangen, entweder überhaupt nicht oder nur in geringsten Spuren vorhanden seir. Bei der technischen Durchführung der bekannten Syntheseverfahren zur Herstellung von 1-Aryloxy-3-amino-2-propanolen, beispielsweise durch Umsetzung eines entsprechenden 1-Aryloxy-2,3-epoxy-propans oder eines 1-Aryloxy-3-("leaving group" - substituierten)-2-propanols mit einem den gewünschten Substituenten enthaltenden Amin (vgl. dazu A.F.Growther et al. J.Med. Chem. 1971, Vol. 14, Nr. 6, Seiten 511 - 513), entstehen Rohprodukte, die Verunreinigungen enthalten, deren Abtrennung immer wieder auf Schwierigkeiten stößt. Es besteht daher nach wie vor Bedarf nach neuen und technisch einfach durchführbaren Reinigungverfahren für Aryloxypropanolamine, die insbesondere die restlose Entfernung von größeren Mengen an Verunreinigungen und Nebenprodukten, die bei der Synthese entstehen, auf einfache Art und Weise ermöglichen sollen.

Es wurde nun unerwarteterweise gefunden, daß Aryloxy-propanolamine mit Diphenylessigsäure neue, gut kristallisierende Salze bilden, welche die kristalline Abscheidung der Aryloxypropanolamine aus Lösungen der bei der Herstellung anfallenden Rohprodukte ermöglichen und dabei einen ausgezeichneten Reinigungserfolg gewährleisten. Aus diesen Diphenylacetaten können die Aryloxypropanolamine mit dem für die medizinische Anwendung erforderlichen Reinheitsgrad in einfacher Weise regeneriert oder in pharmazeutisch verträgliche Salze übergeführt werden.

Gegenstand der Erfindung sind demnach Salze von Aryloxy-propanolaminen mit Diphenylessigsäure der allgemeinen Formel I

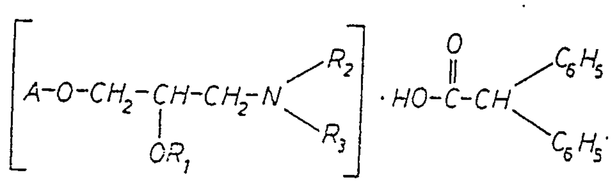

(I)

in der

A

a) einen substituierten Phenylrest der allgemeinen Formel II

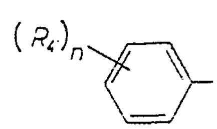

(II)

worin n für eine ganze Zahl von 1 bis 4 steht, die Substituenten $R_4$ entweder gleich oder verschieden sind und unabhängig voneinander für Niederalkyl, C-2- oder C-3-Niederalkenyl, $C_5$ oder $C_6$-Cyclo-alkyl, Niederalkoxy, C-2- oder C-3-Niederalkenoxy, Cycloalkylalkoxy, Tetrahydrofuranyl-methoxy, Niederalkoxyniederalkyl, Niederalkanoyl, Halogen, Hydroxy, Cyano, Carbonamido, Acyloxy, Reste der Formel III oder IV

$$-O-CH_2-X \quad (III) \quad \text{oder} \quad -(CH_2)_m-X \quad (IV)$$

stehen,
wobei X in den Formeln III und IV wiederum Carbamoyl, Alkylcarbamoyl, Dialkylcarbamoyl, Ureido, N'-Alkylureido, N'-Cycloalkylurei do, N,N'-Dialkylureido, N' ,N'-Dialkylureido oder Alkoxycarbonylamino und m in der Formel IV Null oder eine ganze Zahl von 1 bis 3 darstellen,
b) den 1-Naphtyl-2, 2-Naphtyl-, 6,7-Dihydroxy-1-naphtyl, 5-Hydroxy-1-naphtyl-, 5,6,7,8-Tetrahydro-6,7-dihydroxy-1-naphtyl-, 5,6,7,8-Tetrahydro-5-oxo-1-naphtyl-, 9-Fluorenon-4-yl oder Inden-4-ylrest, oder
c) einen Indolyl-, Methylindolyl-, 2-Hydroxy-chinolin , Carbazolyl-oder 4-Morpholinyl-2,1,3-thiadiazolylrest bedeutet,
$R_1$ Wasserstoff oder einen geradkettigen oder verzweigten $C_2$ bis $C_5$-Alkanoyl-oder Aroylrest,
$R_2$ Wasserstoff und
$R_3$ einen verzweigten $C_3$ bis $C_6$-Alkylrest, einen unsubstituierten oder durch Hydroxy oder Alkoxy substituierten, geradkettigen oder verzweigten Phenylniederalkyl-oder Indolylniederalkylrest, einen Rest der Formel V

$$-(CH_2)_l-Y \qquad (V),$$

wobei in der Formel V 1 für eine ganze Zahl 1 oder 2 und Y für einen N'-Phenylureido, N'-Pentamethylenureido-, N'-3"-Oxapentamethylenureido-oder einen unsubstituierten oder substituierten Phenylacetyl-aminorest steht, oder
$R_2$ und $R_3$ unter sich verbunden und zusammen mit dem anliegenden Stickstoffatom einen unsubstituierten oder substituierten 5- oder 6-gliedrigen gesättigten heterocyclischen Rest bedeuten.

Wenn A in der Formel I einen substituierten Phenylrest der allgemeinen Formel II darstellt, sind der oder die Substituenten $R_4$ in der Bedeutung Niederalkyl Ethyl, Propyl, Isopropyl, Butyl, Isobutyl, tert.Butyl oder insbesondere Methyl, in der Bedeutung 2-oder 3-Niederalkenyl 2- oder 3-Butenyl, 1- oder 2-Methallyl oder vorzugsweise Allyl (2-Propenyl) und in der Bedeutung $C_5$ - oder $C_6$-Cycloalkyl Cyclopentyl oder Cyclohexyl. Niederalkoxy bedeutet Ethoxy, Propoxy, Isopropoxy, Butoxy, Isobutoxy oder insbesondere Methoxy, 2- oder 3-Niederalkenoxy bedeutet 2- oder 3-Butenyloxy oder insbesondere Allyloxy (2-Propenyloxy) und Niederalkoxyniederalkyl bedeutet 2-Ethoxy-, 2-Propoxy- oder 2-Butoxy-ethyl, 2-oder 3-Methoxypropyl, 2- oder 3-Ethoxypropyl oder vorzugsweise 2-Methoxyethyl. $R_4$ in der Bedeutung als Niederalkanoyl ist Propionyl, Butyryl oder vorzugsweise Acetyl und in der Bedeutung als Halogen Fluor, Brom, Jod oder insbesondere Chlor.

Der Ausdruck Carbonamido in $R_4$ steht für den Rest eines aliphatischen Carbonsäureamids, der durch Wegfall eines Wasserstoffs aus der $NH_2$-Gruppe des Amids gebildet wird, vorzugsweise für den Acetamido-, Propionamido-oder Butyramidorest. Acyloxy bedeutet vorzugsweise den Acetoxyrest.

Wenn $R_4$ Reste der Formeln III oder IV darstellt, bedeutet in diesen Formeln der Ausdruck Carbamoyl bzw. Alkylcarbamoyl oder Dialkylcarbamoyl in X die Gruppe $-CO-NH_2$ bzw. eine solche Gruppe, in der ein oder beide Wasserstoffatome des $-NH_2$-Restes dieser Gruppe durch Niederalkyl mit der obigen Bedeutung ersetzt sind, z.B. Methylcarbamoyl, Ethylcarbamoyl, Dimethylcarbamoyl, Diethylcarbamoyl. Als Ureidorest bzw. substituierter Ureidorest steht X in den Formeln III und IV für die Gruppe $-CO-NH_2$ bzw. eine solche Gruppe, in der ein oder zwei Wasserstoffe dieser Gruppe in der durch die Notierung am Stickstoff bezeichneten Stelle durch Niederalkyl oder Cycloalkyl jeweils mit der oben angegebenen Bedeutung ersetzt sind, z.B. N'-Methylureido, N,N'-Dimethylureido, N'-Ethylureido, N'-Isopropylureido, N'-Cyclohexylureido, N',N'-Dimethylureido oder insbesondere N',N'-Diethylureido.

X in der Bedeutung Alkoxycarbonylamino steht für einen Niederalkylcarbamatrest $NH-CO-NH_2$ mit der für Niederalkyl oben angegebenen Bedeutung.

Unter A als Indolyl ist ein Rest des 2,3-Benzpyrrols, und als Carbazolyl ein Rest des Dibenzopyrrols zu verstehen.

$R_1$ ist in der Bedeutung als geradkettiger oder verzweigter $C_2$ bis $C_5$-Alkanoylrest beispielsweise Propionyl, Butyryl, Isobutyryl, vorzugsweise Acetyl oder 2,2-Dimethylpropionyl (Pivaloyl) und in der Bedeutung als Aroylrest beispielsweise substituiertes oder unsubstituiertes Benzoyl.

$R_3$ ist in der Bedeutung verzweigter $C_3$ bis $C_6$-Alkylrest vorzugsweise Isopropyl, tert-Butyl, oder tert-Pentyl und in der Bedeutung unsubstituierter oder durch Hydroxy oder Alkoxy substituierter geradkettiger oder verzweigter Phenylniederalkyl-oder Indolylniederalkylrest vorzugsweise der 2-Phenyläthyl-, 2-(3,4-Dimethoxyphenyl)ethyl-oder 2-Indolyl-1,1-dimethylethylrest. Y steht in der Formel V als N′-Phenylureido, bzw. N′-Pentamethylenureido oder N′-3″-Oxapentamethylenureido für einen Ureidorest, in dem am endständigem N-Atom ein Wasserstoff durch Phenyl ersetzt ist, bzw. einen Ureidorest, bei dem das endständige N-Atom zusammen mit der Methylenkette einen Piperidino-oder Morpholinorest bildet und als unsubstituierter oder substituierter Phenylacetylaminorest für eine Gruppe der Formel $C_6H_5$-$CH_2$-CO-NH wobei am Phenylrest dieser Gruppe ein oder mehrere Wasserstoffatome beispielsweise durch Hydroxy substituiert sein können.

Wenn $R_2$ und $R_3$ unter sich verbunden sind und zusammen mit dem anliegenden Stickstoffatom einen unsubstituierten oder substituierten fünf- oder sechsgliedrigen gesättigten heterocyclischen Rest bilden, kann dieser neben dem Stickstoff kein oder noch ein weiteres Heteroatom aufweisen. Fünf- oder sechsgliedrige gesättigte heterocyclische Ringe ohne weiters Heteroatom sind beispielsweise Pyrrolidino oder Piperidino. Fünf- oder sechsgliedrige Ringe, welche ein zusätzliches Heteroatom enthalten, umfassen solche Ringe, in welchen die zusätzliche Heterogruppe NH, N-Niederalkyl, N-Phenyl, O oder S ist, wie Pyrazolidino, Morpholino, Thiomorpholino, Piperazino, N-Niederalkyl-oder N-Phenyl-piperazino, beispielsweise N-(2-Methoxyphenyl)-piperazino.

Aufgrund ihrer ausgezeichneten Kristallistionstendenz und des besonders ausgeprägten Reinigungseffekts sind die neuen Salze der allgemeinen Formel I von folgenden Aryloxy-propanolaminen besonders bevorzugt:

N′-(3-Acetyl-4-(3-((1,1-dimethylethyl)amino)-2-hydroxypropoxy)phenyl) -N,N-diethylharnstoff (Celiprolol)
1-((1-Methylethyl)amino)-3-(1-naphtyloxy)-2-propanol (Propranolol)
1-(2,3-Dimethylphenoxy)-3-((1,1-dimethylethyl)amino-2-propanol (Xibenol)
1-(4-(2-Methoxyphenyl)-piperazin-1-yl)-3-((3,4,5-trimethoxy)phenoxy)-2-propanol (Enciprazin)
1-(2,5-Dichlorphenoxy)-3-((1,1-dimethylethyl)amino)-2-propanol (Cloranolol)
1-(2-Chlor-5-methylphenoxy)-3-((1,1-dimethylethyl)amino)-2-propanol (Bupranolol)
1-(4-(2-Methoxyethyl)phenoxy)-3-((1-methylethyl)amino)-2-propanol (Metoprolol)
4-(2-Hydroxy-3-((1-methylethyl)amino)propoxy)phenylacetamid (Atenolol)
N-(3-Acetyl-4-(2-hydroxy-3-((1-methylethyl)amino)propoxy)phenyl)acetamid (Diacetolol)
1-((1-Methylethyl)amino)-3-(2-(2-propenyloxy)phenoxy)-2-propanol (Oxprenolol)
1-((1,1-Dimethylethyl)amino)-3-(2-((tetrahydro-2-furanyl)methoxy)phenoxy) -2-propanol (Bufetolol)
1-(3-Methylphenoxy)-3-((2-(3,4-dimethoxyphenyl)ethyl)amino)-2-propanol (Bevantolol)
2-(3-((1,1-Dimethylethyl)amino)-2-hydroxypropoxy)benzonitril (Bunitrolol)
N-(3-Acetyl-4-(2-hydroxy-3-((1-methylethyl)amino)propoxy)phenyl)butyramid (Acebutolol)
1-((1,1-Dimethylethyl)amino)-3-((4-morpholinyl)-2,1,3-thiadiazol-5-yl)-2-propanol (Timolol)
N-(2-((3-(2-Cyanophenoxy)-2-hydroxy-propyl)amino)ethyl)-2-(4-hydroxphenyl)-acetamid (Epanolol)
2-(2-Hydroxy-3-((2-(1H)-indol-3-yl-1,1-dimethylethyl)amino)-propoxy) benzonitril (Bucindolol)
5-(3-(1,1-Dimethylethyl)amino)-2-hydroxy-propoxy)-3,4-dihydro-1- (2H)-naphtalinon (Bunolol)
2-(3-((1,1-Dimethylpropyl)amino)-2-hydroxy-propoxy)-benzonitril(Penirolol)
1-(2,4-Dichlorphenoxy)-3-(3,4-dimethoxy-beta-phenylethylamino)-2-propanol
4-(2-Hydroxy-3-((1-methyl-3-phenylpropyl)-amino)-propoxy)phenyl-acetamid
N-(2-((3-(2-Cyanophenoxy-2-hydroxy-propyl)amino)ethyl)-N'-phenylharnstoff.

Die neuen Salze der allgemeinen Formel I werden hergestellt, indem man ein Aryloxy-propanolamin der allgemeinen Formel VI

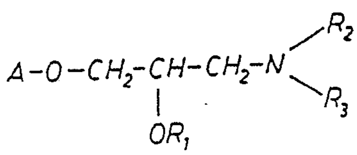

(VI)

in der A,
$R_1$, R2 und $R_3$ die unter Formel I angegebene Bedeutung haben, in einem gegenüber den Reaktionspartnem inerten Lösungsmittel oder Lösungsmittelgemisch bei Temperaturen von Raumtemperatur bis zur Siedetemperatur des Lösungsmittels oder der niedrigstsiedenden Lösungsmittelkomponente mit einer mindestens stöchiometrischen Menge an Diphenylessigsäure umsetzt, das sich bei Raumtemperatur oder bevorzugt in der Kälte abscheidende Diphenylacetat der Formel I aus dem bei der Umsetzung erhaltenen Reaktionsgemisch abtrennt und gegebenenfalls aus einem organischen Lösungsmittel umkristallisiert.

Beispielsweise werden die Salze der allgemeinen Formel I durch direkte Umsetzung von Lösungen eines Aryloxy-propanolamins der Formel VI und der Diphenylessigsäure in geeigneten Lösungsmitteln erhalten. Als solche Lösungsmittel seien u.a. genannt aliphatische Alkohole, wie Methanol, Ethanol,

Propanol, Isopropanol, Butanol und dergleichen; chlorierte Kohlenwasserstoffe, wie Methylenchlorid, Chloroform, Tetrachlorkohlenstoff und dergleichen; aliphatische Carbonsäureamide, Carbonsäureester oder Carbonsäurenitrile, wie Dimethylformamid, Dimethylacetamid, Ethylacetat, Acetonitril und dergleichen, aliphatische Ketone wie Aceton, Methylethylketon und dergleichen; Gemische, welche diese Lösungsmittel enthalten oder Gemische dieser Lösungsmittel mit Wasser. Ein besonders bevorzugtes Lösungsmittel für die erfindungsgemäße Umsetzung ist Aceton.

Innerhalb der oben angegebenen Grenzen kann die Umsetzungstemperatur nahezu beliebig gewählt werden, wobei die Umsetzung aber bevorzugt bei Raumtemperatur oder Temperaturen, die knapp darüber oder darunter liegen, durchgeführt wird.

Die erfindungsgemäßen Salze der Formel I werden aber auch durch Umsalzung, z.B. durch Umsetzung von entsprechenden Salzen eines Aryloxy-propanolamins der Formel VI, wie den Hydrohalogeniden, vorzugsweise dem Hydrochlorid, oder den Sulfaten mit geeigneten Salzen der Diphenylessigsäure, beispielsweise Alkalimetall-, vorzugsweise Natrium- oder Ammoniumsalzen erhalten. Hiezu werden die Ausgangsmaterialien in geeigneten Lösungsmitteln gelöst und einander zugesetzt. Wesentlich ist ein ausreichender Löslichkeitsunterschied zwischen dem als Ausgangsmaterial verwendeten Salz des Aryloxypropanolamins und dem Diphenylacetat der Formel I, wobei das als Nebenprodukt entstehende anorganische Salz, z.B. Natriumchlorid, leicht abtrennbar sein soll. Geeignete Lösungsmittel für die Umsalzung sind beispielsweise Wasser, niedere aliphatische Alkohole wie Methanol, Ethanol oder aliphatische Ketone, wie Aceton, Methylethylketon, Carbonsäureamide, Carbonsäurenitrile, wie Dimethylformamid, Dimethylacetamid, Acetonitril, Gemische welche diese Lösungsmittel enthalten oder Gemische dieser Lösungsmittel mit Wasser.

Die erfindungsgemäßen Diphenylacetate der allgemeinen Formel I haben eine außergewöhnlich hohe Kristallisationstendenz und scheiden sich auch aus Lösungen der Aryloxy-propanolamine, die Verunreinigungen und/oder Nebenprodukte organischer oder anorganischer Natur enthalten, ohne Einschlüsse von Fremdstoffen oder Mischkristallbildung in reiner Form ab. Sie eignen sich daher hervorragend zur kristallinen Abscheidung der Aryloxy-propanolamine mit einem hohen Reinheitsgrad aus Lösungen ihrer Rohprodukte, während Verunreinigungen und Nebenprodukte in Lösung bleiben.

Die Diphenylacetate der Formel I werden nach dem Abtrennen aus dem Reaktionsgemisch meistens in so reiner Form erhalten, daß weitere Reinigungsschritte, beispielsweise durch Umkristallisieren oder Umfällen, nicht mehr notwendig sind. In den wenigen Fällen, in denen die Diphenylacetate nicht in dem Reinheitsgrad erhalten werden, der für ihre weitere Verwendung zur Herstellung von Aryloxypropanolaminen oder von deren pharmazeutisch verträglichen Salzen für medizinische Zwecke notwendig ist, können sie auf einfache Weise durch Umkristallisieren aus organischen Lösungsmitteln nochmals gereinigt werden. Als solche Lösungsmittel eignen sich beispielsweise niedere aliphatische Alkohole, wie Methanol, Ethanol, Propanol, Isopropanol, Butanol und dergleichen aliphatische Ketone, wie Aceton, Methylethylketon und dergleichen, Carbonsäureester oder Carbonsäurenitrile, wie Ethylacetat, Acetonitril und dergleichen.

Es ist daher nicht notwendig, zur Herstellung der Diphenylacetate der allgemeinen Formel I die Aryloxypropanolamine der Formel VI in reiner Form einzusetzen.

Ein besonderer Vorteil der erfindungsgemäßen Salze besteht darin, daß diese auch dann in reiner Form erhalten werden, wenn man zu ihrer Herstellung das Rohprodukt eines Aryloxy-propanolamins einsetzt, wie es auf einem für diese Verbindungen üblichen Syntheseverfahren nach der Abtrennung aus dem Reaktionsgemisch ohne weiteren Reinigungschritt erhalten wird. Ein weiterer Vorteil ist darin gelegen, daß reine kristalline Salze der Formel I sogar aus nicht kristallisierbaren, öligen Rohprodukten erhalten werden und deren Reinigung mit hoher Ausbeute ermöglichen.

Daher ist es möglich, die Herstellung der Aryloxy-propanolamine und die anschließende Umwandlung in die Diphenylacetate der allgemeinen Formel I vorteilhafterweise auch in einer Eintopfreaktion durchzuführen, indem man die basischen Aryloxypropanolamine unmittelbar nach ihrer Herstellung durch Zugabe von Diphenylessigsäure in die Salze überführt.

Die erfindungsgemäßen Salze eignen sich besonders zur Reinigung von Rohprodukten von Aryloxypropanolaminen, wie diese durch Umsetzung eines 1-Aryloxy-2,3-epoxypropans der Formel VII

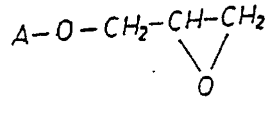

(VII)

,

oder einer Verbindung der Formel VIII

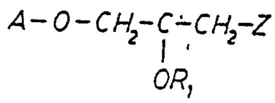

(VIII)

mit einem Amin der Formel IX

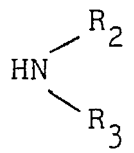

(IX)

erhalten werden, wobei in den obigen Formeln A, $R_1$, $R_2$ und $R_3$ die unter Formel I angegebene Bedeutung haben und Z für eine leicht abspaltbare Abgangsgruppe steht. Solche Umsetzungen sind beispielsweise in der DE-PS-2,458.624, in der DE-OS-3,039.848 oder in J.Med.Chem. 1971, Vol. 14 Nr. 6, Seiten 511 - 513 beschrieben.

Die oben angegebene Umsetzung ist ein sehr einfaches, wirtschaftliches und häufig angewandtes Verfahren zur Herstellung von Aryloxy-propanolaminen. Insbesondere bei der Herstellung von 1-Aryloxy-3-amino-2-propanolen mit einer endständigen sekundären Aminogruppe, die wegen ihrer therapeutischen Bedeutung der Zahl nach überwiegen und die auf dem genannten Syntheseweg durch Umsetzung einer Verbindung der Formel VII oder VIII mit einem primären Amin der Formal IX erhalten werden können, werden tertiäre Amine mit zwei Aryloxy-propanolresten in größeren Mengen als unerwünschte Nebenprodukte gebildet. Die Reinigung von Rohprodukten aus einer solchen Synthese bereitet nach dem Stand der Technik besondere Schwierigkeiten.

Zur Vereinfachung der Reinigung wurde bisher die als Konkurrenzreaktion ablaufende Bildung von Nebenprodukten in Form der tertiären Amine durch Verwendung eines sehr großen, beispielsweise bis zu 10-fachen, Aminüberschusses zurückgedrängt oder durch Einsatz von Aminen, die zusätzlich eine leicht abspaltbare Schutzgruppe tragen, gänzlich umgangen. Beide Methoden sind aber mit erheblichen Nachteilen behaftet und erübrigen nicht zur Gänze weitere Reinigungsoperationen, die zur Gewinnung der Verbindungen mit dem für medizinische Zwecke notwendigen Reinheitsgrad erforderlich sind. So kann das über-schüssig eingesetzte Amin meistens nur mit entsprechenden Verlusten wiedergewonnen werden, wobei die bei dieser Arbeitsmethode notwendigerweise auftretende Verdünnung des Reaktionsansatzes zusätzlich eine ungünstige Raum-Zeitausbeute bewirkt. In jenen Fällen, in denen das überschüssige Amin nur schwer flüchtig ist, ist diese Methode gänzlich unbrauchbar.

Beim Einsatz von geschützten Aminen wiederum muß der meist ohnehin vielstufigen Synthese der Aryloxy-propanolamine ein weiterer Verfahrensschritt zur Entfernung der Schutzgruppe angefügt werden, sodaß auch diese Methode bei der Herstellung der Verbindungen in technischem Maßstab nur ungern angewandt wird.

Ein überraschender Vorteil der erfindungsgemäßen Salze besteht nun darin, daß diese aus Rohprodukten, die neben tertiären Aminen und/oder anderen Verunreinigungen beispielsweise nur etwa 60 % des gewünschten Aryloxy-propanolamins enthalten, in reiner Form abgetrennt werden können und eine Reinigung solcher Rohprodukte ermöglichen, was mit den bekannten Reinigungsverfahren nur sehr schwierig zu erreichen ist. Dadurch ist es möglich das oben näher beschriebene Syntheseverfahren für Aryloxy-propanolamine auf wirtschaftliche und vorteilhaftere Weise mit äquivalenten Mengen oder nur geringen, beispielsweise zwei- bis dreifachen, Überschüssen der Aminkomponente durchzuführen, ohne daß die Reinigung der dabei erhaltenen Rohprodukte auf unüberwindliche Schwierigkeiten stößt.

Proben von Salzen der allgemeinen Formel I, die aus Rohprodukten von Aryloxy-propanolaminen der Formel VI hergestellt wurden, weisen einen außerordentlich hohen Reinheitsgrad auf. In solchen Proben werden bei Untersuchungen mit chromatographischen Methoden, wie Dünnschicht-Chromatographie oder Hochdruckflüssigkeitschromatographie, Schmelzpunktuntersuchungen oder spektroskopischen Methoden Nebenprodukte und Verunreinigungen entweder überhaupt nicht oder nur in geringsten Mengen gefunden, die die weitere Verwendung der Salze der Formel I zur Herstellung von Präparaten für medizinische Zwecke in keiner Weise beeinträchtigen.

Aus den erfindungsgemäßen Diphenylacetaten der allgemeinen Formel I können die Aryloxy-propanolamine auf übliche Weise wiedergewonnen oder in ihre pharmazeutisch verträglichen Säureadditionssalze überführt werden.

Ein weiterer Gegenstand der vorliegenden Erfindung betrifft daher die Verwendung der auf einem der genannten Wege erhaltenen reinen Salze der allgemeinen Formel I zur Herstellung von Aryloxypropanolaminen der allgemeinen Formel VI oder von deren pharmazeutisch verträglichen Salzen mit dem für die medizinische Anwendung notwendigen Reinheitsgrad.

Hiezu werden die Aryloxypropanolamine der allgemeinen Formel VI aus den Diphenylacetaten der allgemeinen Formel I in an sich bekannter Weise durch Zusatz von Basen wiedergewonnen und dann gewünschtenfalls in pharmazeutisch verträglichen Säureadditionssalze überführt. Das kann beispielweise dadurch geschehen, daß man das Diphenylacetat in einem organischen, mit Wasser nicht mischbaren Lösungsmittel löst und dann die erhaltene Lösung mit einer verdünnten wässerigen Lösung einer Base, wie Natronlauge, Kalilauge und dergleichen behandelt. Die organische Phase kann anschließend abgetrennt, getrocknet und im Vakuum eingedampft werden, wobei das Aryloxypropanolamin in reiner Form zurückbleibt. Die basisch reagierenden Verbindungen der Formel VI können dann gewünschtenfalls mit Säuren, wie diese beispielsweise in J. Pharm. Sei. 66, 1 -16, zur Herstellung von therapeutisch verträglichen Salzen beschrieben sind, in ihre Säureadditionssalze überführt werden. Zur Salzbildung geeignet sind beispielsweise starke Mineralsäuren wie Chlorwasserstoff-, Bromwasserstoff-, Jodwasserstoff-, Fluorwasserstoffsäure, Schwefelsäure, Phosphorsäure, Salpetersäure und Perchlorsäure, aliphatische, alicyclische, aromatische, heterocyclische Carbonsäuren oder Sulfonsäuren wie Ameisensäure, Essigsäure, Propionsäure, Bernsteinsäure, Fumarsäure, Maleinsäure, Glykolsäure, Milchsäure, Apfelsäure, Benzoesäure, Salicylsäure, Nikotinsäure, Cyclohexylsulfonsäure, Methansulfonsäure oder Amidosulfonsäuren und dergleichen.

Auf besonders vorteilhafte und einfache Weise können die Diphenylacetate der allgemeinen Formel I in an sich bekannter Weise durch Umsalzen direkt in pharmazeutisch verträgliche Säureadditionssalze übergeführt werden, wobei das Diphenylacetat-Anion durch ein pharmazeutisch verwendbares Anion ersetzt wird. Zur Umsalzung wird das Diphenylacetat beispielsweise in einem organischen Lösungsmittel gelöst oder suspendiert und mit einer das gewünschte Anion enthaltenden Säure, vorzugsweise mit einer starken Mineralsäure, wie Chlorwasserstoff-, Bromwasserstoff-, Jodwasserstof-, Fluorwasserstoffsäure, Schwefelsäure, Phosphorsäure, Salpetersäure, Perchlorsäure und dergleichen, behandelt. Wesentlich für die Umsalzung ist die Herstellung eines ausreichenden Löslichkeitsunterschiedes durch Wahl eines geeigneten Lösungsmittels, in dem die freigesetzte Diphenylessigsäure gut löslich ist, während das Salz des Aryloxypropanolamins, welches das Anion der gewünschten Säure enthält, in diesem Lösungsmittel nur schlecht löslich sein soll und daher ausfällt.

Geeignete Lösungsmittel sind beispielsweise aliphatische Ketone, wie Aceton, Methylethylketon und dergleichen, aliphatische Alkohole wie Propanol, Isopropanol, Butanol, Isobutanol, tert.-Butanol, Amylalkohol und dergleichen, Carbonsäureester oder Carbonsäurenitrile, wie Ethylacetat, Butylacetat, Acetonitril und dergleichen.

Ein weiterer Vorteil der Erfindung besteht darin, daß sowohl bei der Wiedergewinnung der Aryloxypropanolamine aus den Diphenylacetaten der Formel I mittels einer Base als auch bei der Umsalzung die freigesetzte Diphenylessigsäure in einfacher Weise meistens in nahezu quantitativen Ausbeuten zurückgewonnen und erneut verwendet werden kann.

Die folgenden Beispiele erläutern die Erfindung näher, wobei die Ausbeuten an Diphenylacetat der Formel I naturgemäß in Abhängigkeit vom Reinheitsgrad der eingesetzten Rohprodukte unterschiedlich ausfallen.

Beispiele 1 - 6:

N′-(3-Acetyl-4-(3-((1,1-dimethylethyl)amino)-2-hydroxypropoxy)phenyl)-N,N-diethylharnstoff (Celiprolol)-diphenylacetat
A: 3-Acetyl-4-(N′,N′-diethylureido)phenyl
$R_1$: H
$R_2$: H
$R_3$: 1,1-Dimethylethyl

Beispiel 1:

524.5 g des nach einem der unten angegebenen Syntheseverfahren ohne weiteren Reinigungsschritt erhaltenen Rohprodukts von N′-(3-Acetyl-4-(3-((1,1-dimethylethyl)amino)-2-hydroxypropoxy)phenyl)-N,N-diethylharnstoff (Wassergehalt 4.67 %), entsprechend 500 g des wasserfreien Rohprodukts, werden in 3500 ml Aceton bei 25° C gelöst, eine vorhandene Trübung abgetrennt, mit 300 ml Aceton nachgewaschen, das Filtrat auf 18° C abgekühlt und mit einer Lösung von 282 g Diphenylessigsäure (Wassergehalt 0.82 %) in

1200 ml Aceton versetzt, wobei das Diphenylacetat der eingesetzten Base auszukristallisieren beginnt. Zur Vervollständigung der Kristallisation wird der Ansatz 4 Stunden mit kaltem Wasser gekühlt, das ausgefallene Kristallisat abgesaugt, mit 1500 ml Aceton nachgewaschen und an der Luft getrocknet.
Ausbeute: 740 g (94.9 % der Theorie)
MFp. 140 - 142° C

Das so erhaltene Diphenylacetat ist analysenrein und zeigt im Dünnschichtchromatogramm in folgenden Laufmitteln einen einheitlichen Spot:
Laufmittel I: Benzol/Aceton/Eisessig/Wasser; 40/50/30/20; v/v/v/v
Laufmittel II: Ethylacetat/Ethanol/2n $NH_4OH$; 50/25/20; v/v/v
Entwicklung: UV

Beispiel 2:

10.0 g eines Rohprodukts von N'-(3-Acetyl-4-(3-((1,1-dimethylethyl)amino)-2-hydroxypropoxy)phenyl)-N,N-diethylharnstoff-hydrat werden in 150 ml Acetonitril gelöst und mit einer Lösung von 5,6 g Diphenylessigsäure in 50 ml Acetonitril versetzt.
Das ausgefallene Kristallisat wird nach 5-stündigem Stehen bei Raumtemperatur abgesaugt.
Ausbeute: 14.3 g (96.1 % der Theorie)
Das erhaltene Diphenylacetat ist analysenrein.
MFp: 140 - 142° C.

Beispiel 3:

10.0 g eines Rohprodukts von N'-(3-Acetyl-4-(3-((1,1-dimethylethyl)amino)-2-hydroxypropoxy)phenyl)-N,N-diethylharnstoffhydrat werden in 40 ml Ethanol gelöst, mit einer Lösung von 5,6 g Diphenylessigsäure in 40 ml Ethanol versetzt, das Reaktionsgemisch über Nacht bei 0° C eingekühlt und das ausgefallene Kristallisat abgesaugt.
Ausbeute: 7.1 g (47,7 % der Theorie)
Das erhaltene Diphenylacetat ist analysenrein.
MFp.: 140 - 142° C

Beispiel 4:

2.0 g eines Rohprodukts von N'-(3-Acetyl-4-(3-((1,1-dimethylethyl)amino)-2-hydroxypropoxy)phenyl)-N,N-diethylharnstoff-hydrat werden in 50 ml Ethylacetat gelöst, 1,12 g Diphenylessigsäure zugesetzt und das ausgefallene Kristallisat nach einer Stunde bei Raumtemperatur abgesaugt.
Ausbeute: 2.87 g (96,4 % der Theorie)
Das erhaltene Diphenylacetat ist analysenrein
MFp.: 140 - 142° C

Beispiel 5:

2.0 g eines Rohprodukts von N'-(3-Acetyl-4-(3-((1,1-dimethylethyl)amino)-2-hydroxypropoxy)-phenyl)-N,N-diethylharnstoff-hydrat werden in 5.0 ml Methanol gelöst, 1.12 g Diphenylessigsäure zugegeben, die klare Lösung mit 5 ml Wasser versetzt und das ausgefallene Kristallisat nach 2 Stunden bei Raumtemperatur abgesaugt.
Ausbeute: 2.4 g (80.6 % der Theorie)
Das erhaltene Diphenylacetat ist analysenrein
MFp.: 140 - 142° C

Beispiel 6:

2.0 g eines Rohprodukts von N'-(3-Acetyl-4-((1,1-dimethylethyl)amino)-2-hydroxypropoxy)-phenyl)-N,N-diethylharnstoff-hydrat werden in 20 ml n-Butanol gelöst, 1.12 g Diphenylessigsäure zugegeben und das entstandene Kristallisat nach Stehen über Nacht bei Raumtemperatur abgesaugt.
Ausbeute: 2.5 g (83.9 % der Theorie)
Das erhaltene Diphenylacetat ist analysenrein.
MFp.: 140 - 142° C

Herstellung des als Ausgangsmaterial verwendeten Rohprodukts:

Variante A:

365.4 g tert-Butylamin (4.99 Mol) werden mit 261.8 ml $H_2O$ vermischt und auf Raumtemperatur abgekühlt. Sodann werden 750 g N′-(3-Acetyl-4-(2,3-epoxypropoxy)-phenyl)-N,N-diethylharnstoff (2,45 Mol) zugesetzt und bei einer Temperatur von 24 bis 27° C 8 Stunden zur Reaktion gebracht.
Das Reaktionsgemisch wird hierauf mit 750 ml Aceton versetzt, vermischt und bei maximal 25° C langsam mit insgesamt 1500 ml Wasser versetzt, angeimpft, über Nacht bei 3° C stehen gelassen und die ausgefallene Base abzentrifugiert. Das Rohprodukt wird in einer Mischung von 200 ml Aceton und 1800 ml Wasser aufgeschlämmt, zentrifugiert und an der Luft getrocknet.

Ausbeute an rohem N′-(3-Acetyl-4-(3-((1,1-dimethylethyl)amino)-2-hydroxypropoxy)phenyl)-N,N-diethylharnstoff-hydrat: 902.2 g (92.7 % der Theorie)

Dieses Produkt ist im Dünnschichtchromatogramm uneinheitlich und zeigt neben dem Hauptspot mehrere auf Verunreinigungen zurückzuführende Spots mit größerem oder kleinerem Rf-Wert.

Variante B:

1.0 g N′-(3-Acetyl-4-(3-brom-2-hydroxy-propoxy)phenyl)-N,N-diethylharnstoff (0.0026 Mol) werden mit einer Mischung von 0.825 ml tert-Butylamin (0.0078 Mol) und 0.42 ml Wasser versetzt und 30 Stunden bei Raumtemperatur gerührt. Das Reaktionsgemisch wird im Vakuum konzentriert, mit Wasser versetzt und mit Chloroform extrahiert. Nach dem Trocknen mit $Na_2SO_4$ wird die Chloroformlösung im Vakuum abdestilliert. Das als Trockenrückstand verbliebene Rohprodukt des N′-(3-Acetyl-4-(3-((1,1-dimethylethyl)amino)-2-hydroxy-propoxy)phenyl)-N,N-diethylharnstoff wird ohne weitere Reinigung für die Herstellung des Diphenylacetats gemäß den Beispielen 1 - 6 verwendet.

Variante C:

1.0 g N′-(3-Acetyl-4-(3-chlor-2-hydroxy-propoxy)phenyl)-N,N-diethylharnstoff (0.0029 Mol) werden mit einer Mischung von 0.924 ml tert.-Butylamin (0.0087 Mol) und 0.46 ml Wasser 13 Tage bei Raumtemperatur zur Reaktion gebracht.
Die Aufarbeitung erfolgt wie in Variante B und der dabei erhaltene Trockenrückstand wird als Rohprodukt zur Herstellung des Diphenylacetats gemäß den Beispielen 1 -6 verwendet.

Variante D:

2.0 g (0.0042 Mol) N′-(3-Acetyl-4-(3-tosyloxy-2-hydroxy-propoxy)-phenyl)-N,N-diethylharnstoff werden mit einer Mischung von 1.0 ml tert.-Butylamin (0.0095 Mol) und 1.0 ml Wasser 18 Stunden bei Raumtemperatur zur Reaktion gebracht.
Das Reaktionsgemisch wird in Chloroform aufgenommen, zweimal mit Wasser gewaschen, die organische Phase mit $Na_2SO_4$ getrocknet und abdestilliert. Der dabei erhaltene Trockenrückstand wird als Rohprodukt zur Herstellung des Diphenylacetats gemäß den Beispielen 1 - 6 verwendet.

Beispiel 7:

1-((1-Methylethyl)amino)-3-(1-naphtyloxy)-2-propanol (Propranolol)-diphenylacetat

A: 1-Naphtyl
$R_1$: H
$R_2$: H
$R_3$: 1-Methylethyl
6.44 g eines Rohprodukts von 1-((1-Methylethyl)amino)-3-(1-naphtyloxy)-2-propanol werden in 25 ml Aceton bei 45° C gelöst mit 5.27 g Diphenylessigsäure, welche zuvor mit 10 ml Aceton in der Wärme gelöst wurde, versetzt und das Reaktionsgemisch gekühlt. Nach dem Stehen in der Kälte wird das ausgefallene Kristallisat abgesaugt, mit Aceton gewaschen und getrocknet.
Ausbeute 9.3 g (78.97 % der Theorie, bezogen auf das für die Herstellung des Ausgangsmaterials eingesetzte 1-(1-Naphtoxy)-2,3-epoxy-propan).
Das Diphenylacetat zeigt im Dünnschichtchromatogramm einen einheitlichen Spot.
MFp.: 146 - 148.5° C

Herstellung des als Ausgangsmaterial verwendeten Rohprodukts:

2.55 ml (1-Methylethyl)amin (0.0297 Mol) werden mit 1.25 ml $H_2O$ vermischt und hierauf mit 5 g 1-(1-Naphtyloxy)-2,3-epoxy-propan (0.0249 Mol) verrührt und 23 Stunden bei Raumtemperatur zur Reaktion gebracht.
Das Umsetzungsprodukt wird in $CHCl_3$ aufgenommen, mit Wasser ausgeschüttelt, die organische Phase abgetrennt und das Lösungsmittel im Vakuum abgetrennt.
Man erhält so als Trockenrückstand 6.44 g eines Rohprodukts von 1-((1-Methylethyl)amino)-3-(1-naphtyloxy)-2-propanol aus dem die Reinsubstanz als kristallines Diphenylacetat abgeschieden werden kann.

Beispiel 8:

1-(4-(2-Methoxyphenyl)-piperazin-1-yl)-3-((3,4-5-trimethoxy)phenoxy)-2-propanol(Enciprazin)-diphenylacetat
A: 3,4,5-Trimethoxyphenyl
$R_1$: Wasserstoff
$R_2$ und $R_3$ gemeinsam mit dem anliegenden N-Atom: 4-(2-Methoxyphenyl)-piperazin-1-yl
7.3 g eines Rohprodukts von 1-(4-(2-Methoxyphenyl-piperazin-1-yl)-3-((3,4,5-trimethoxy)phenoxy)-2-propanol werden in 35 ml Aceton gelöst, eine Lösung von 3.6 g Diphenylessigsäure in 10 ml Aceton zugesetzt und das ausgefallene Kristallisat nach 5-stündigem Stehen bei 0° C abgetrennt.
Ausbeute 8.5 g (77.19 % der Theorie)
MFp.: 119 - 120.5° C
Das Diphenylacetat zeigt im Dünnschichtchromatogramm in mehreren Systemen einen einheitlichen Spot.
Stationäre Phase: Kieselgel $HF_{60}$
Mobile Phase I: Benzol/Methanol/Aceton; 50/5/25; v/v/v
Mobile Phase II: Benzol/Methanol; 25/50; v/v
Entwicklung: UV

Herstellung des als Ausgangsmaterial verwendeten Rohprodukts:

4.7 g 1-(3,4,5-Trimethoxy)phenoxy-2,3-epoxy-propan (0.0196 Mol) werden in einer Mischung von 1.9 ml Wasser und 1.9 ml Ethanol mit 3.95 g 1-(2-Methoxy)-phenylpiperazin (0.0223 Mol) 4 Stunden bei Raumtemperatur zur Reaktion gebracht. Anschließend wird das Lösungsmittel im Vakuum abgezogen, der Rückstand mit Diethylether versetzt und das ausgefallene Produkt nach Stehen über Nacht abgesaugt.
Man erhält so 7.4 g (90.61 % der Theorie) eines Rohprodukts von 1-(4-(2-Methoxyphenyl-piperazin-1-yl)-3-((3,4,5-trimethoxy)phenoxy)-2-propanol.

Beispiel 9:

1-(2,5-Dichlorphenoxy)-3-((1,1-dimethylethyl)amino)-2-propanol (Cloranolol)-diphenylacetat.
A: 2,5-Dichlorphenyl

$R_1$: H
$R_2$: H
$R_3$: 1,1-Dimethylethyl
8.55 g einer Rohbase von 1-(2,5-Dichlorphenoxy)-3-((1,1-dimethylethyl)amino)-2-propanol in Form eines öligen Produkts werden in 10 ml Aceton gelöst, mit einer Lösung von 6.2 g Diphenylessigsäure in 25 ml Aceton versetzt und das ausgefallene Kristallisat nach 2-stündigem Stehen bei Raumtemperatur abgesaugt.
Ausbeute: 12.4 g (86.8 % der Theorie)
MFp.: 155 - 156,5° C
Das Diphenylacetat zeigt im Dünnschichtchromatogramm in mehreren Systemen einen einheitlichen Spot.
Stationäre Phase: Kieselgel $HF_{60}$
Mobile Phase I: Benzol/Aceton/Methanol/Wasser; 40/50/30/20; v/v/v/v
Mobile Phase II: Benzol/Methanol/$NH_4OH$; 50/12.5/0.1; v/v/v

Herstellung des als Ausgangsmaterial verwendeten Rohprodukts:

6.2 g 1-(2,5-Dichlor)-phenoxy-2,3-epoxy-propan (0.0283 Mol) werden in einer Mischung von 6 ml (1,1-Dimethylethyl)amin (0.057 Mol) und 3 ml Wasser 24 Stunden bei Raumtemperatur zur Reaktion gebracht. Das Reaktionsgemisch wird im Vakuum konzentriert, der Rückstand in Methylenchlorid aufgenommen, die organische Phase mit Wasser gewaschen, mit $Na_2SO_4$ getrocknet und das Lösungsmittel abdestilliert.
Man erhält so 8.55 g eines Rohprodukts von 1-(2,5-Dichlorphenoxy)-3-((1,1-dimethylethyl)amino)-2-propanol in Form eines öligen Rückstandes.

Beispiel 10:

1-(2,3-Dimethylphenoxy)-3-((1,1-dimethylethyl)amino)-2-propanol (Xibenolol)-diphenylacetat:
A: 2,3-Dimethylphenyl
$R_1$: H
$R_2$: H
$R_3$: 1,1-Dimethylethyl
1.6 g der rohen Base von 1-(2,3-Dimethylphenoxy)-3-((1,1-dimethylethyl) amino)-2-propanol in Form eines öligen, nicht kristallisierbaren Produkts werden in 5 ml Aceton gelöst und mit einer Lösung von 1.35 g Diphenylessigsäure in 5 ml Aceton versetzt. Das ausgefallene Kristallisat wird nach 1-stündigem Stehen bei Raumtemperatur abgesaugt.
Ausbeute: 2.5 g (84.7 % der Theorie)
MFp.: 148 - 150° C
Das Diphenylacetat zeigt im Dünnschichtchromatogramm in mehreren Systemen einen einheitlichen Spot.
Stationäre Phase und Laufmittel wie in Beispiel 9
Entwicklung: Besprühen der DC-Platte mit 1%iger $J_2$-Lösung in Ethanol.

Herstellung des als Ausgangsmaterial verwendeten Rohprodukts:

1,2 g 1-(2,3-Dimethyl)-phenoxy-2,3-epoxy-propan werden mit einer Mischung von 1.4 ml (1,1-Dimethylethyl)amin (0.013 Mol) und 0.7 ml Wasser zur Reaktion gebracht. Das Reaktionsgemisch wird in Vakuum konzentriert, der Rückstand in Methylenchlorid aufgenommen, mit Wasser gewaschen und die organische Phase nach dem Trocknen abdestilliert.
Man erhält so 1.69 g eines Rohprodukts von 1-(2,3-Dimethylphenoxy)-3-((1,1-dimethylethyl)amino)-2-propanol in Form eines öligen Rückstands.

Beispiel 11:

1-(2-Cyano)-phenoxy-3-((1,1-Dimethylpropyl)amino)-2-propanol (Penirolol)-diphenylacetat
A: 2-Cyanophenyl
$R_1$: H
$R_2$: H

$R_3$: 1,1-Dimethylpropyl

1.87 g der rohen Base von 1-(2-Cyano)-phenoxy-3-((1,1-Dimethylpropyl)amino)-2-propanol in Form eines öligen, nicht kristallisierbaren Produkts werden in 5 ml Aceton gelöst, eine Lösung von 1.5 g Diphenylessigsäure in 3 ml Aceton zugegeben und das ausgefallene Kristallisat nach 1,5-stündigem Stehen bei Raumtemperatur abgetrennt.

Ausbeute: 2.6 g (76.8 % der Theorie, bezogen auf das für die Herstellung des Ausgangsmaterials eingesetzten 1-(2-Cyano)-phenoxy-2,3-epoxy-propan.

Die Reinheitskontrolle im Dünnschichtchromatogramm ergibt in mehreren Systemen einen einheitlichen Spot.

Stationäre Phase: Kieselgel $HF_{60}$

Mobile Phase: Benzol/Aceton/Eisessig/Wasser; 40/50/30/20; v/v/v/v

Entwicklung: Besprühen mit 1%iger Jod-Lösung in Ethanol

Herstellung des als Ausgangsmaterial verwendeten Rohprodukts:

1.25 g 1-(2-Cyano)-phenoxy-2,3-epoxy-propan (0.0071 Mol) werden mit einer Mischung von 1.24 g (1,1-Dimethylpropyl)amin (0.014 Mol) und 0.8 ml Wasser 22 Stunden bei Raumtemperatur zur Reaktion gebracht. Das Reaktionsgemisch wird im Vakuum konzentriert, der Rückstand in Chloroform aufgenommen, mit Wasser gewaschen, die organische Phase getrocknet und das Lösungsmittel im Vakuum abdestilliert. Man erhält so 1.87 g eines Rohprodukts von 1-(2-Cyano)-phenoxy-3-((1,1-Dimethylpropyl)amino)-2-propanol in Form eines öligen Rückstandes.

Analog zu den in den Beispielen 1 - 11 angegebenen Arbeitsvorschriften wurden aus den Rohprodukten der jeweiligen Aryloxy-propanolamine folgende neue Salze der allgemeinen Formel I hergestellt und auf ihren Reinheitsgrad überprüft:

Beispiel 12:

N-(3-Acetyl-4-(2-hydroxy-3-((1-methylethyl)amino)propoxy)phenyl)butyramid (Acebutolol)-diphenylacetat

A: 2-Acetyl-4-butyramido-phenyl

$R_1$: H

$R_2$: H

$R_3$: 1-Methylethyl

MFp.: 108 - 110° C

Ausbeute: 97,2 % der Theorie

Beispiel 13:

1-((1-Methylethyl)amino)-3-(2-(2-propenyl)phenoxy)-2-propanol (Alprenolol)-diphenylacetat

A: 2-(2-Propenyl)phenyl

$R_1$: H

$R_2$: H

$R_3$: 1-Methylethyl

MFp.: 91 - 94° C

Ausbeute: 75,67 % der Theorie

Beispiel 14:

4-(2-Hydroxy-3-((1-methylethyl)amino)propoxy)phenylacetamid (Atenolol)-diphenylacetat

A: 4-Carbamoylmethyl-phenyl

$R_1$: H

$R_2$: H

$R_3$: 1-Methylethyl

MFp.: 72 - 74.5° C

Ausbeute: 97,5 % der Theorie

Beispiel 15:

1-(3-Methylphenoxy)-3-((2-(3,4-dimethoxyphenyl)ethyl)amino-2-propanol (Bevantolol)-diphenylacetat
A: 3-Methylphenyl
$R_1$: H
$R_2$: H
$R_3$: 2-(3,4-Dimethoxyphenyl)ethyl
MFp.: 146 - 148° C
Ausbeute: 40,61 % der Theorie

Beispiel 16:

2-(2-Hydroxy-3-((2-(1H)-indol-3-yl-1,1-dimethylethyl)amino)propoxy) benzonitril (Bucindolol)-diphenylacetat
A: 2-Cyanophenyl
$R_1$: H
$R_2$: H
$R_3$: -2-((1H)-indol-3-yl)-1,1-dimethylethyl
MFp.: 138 - 140° C
Ausbeute: 56,96 % der Theorie

Beispiel 17:

1-((1,1-Dimethylethyl)amino)-3-(2-((tetrahydro-2-furanyl)methoxy) phenoxy) -2-propanol (Bufetolol)-diphenylacetat
A: 2-((Tetrahydro-2-furanyl)-methoxy)phenyl
$R_1$: H
$R_2$: H
$R_3$: 1,1-Dimethylethyl
MFp.: 125 - 127° C
Ausbeute: 66,26 % der Theorie

Beispiel 18:

2-(3-((1,1-Dimethylethyl)amino)-2-hydroxypropoxy)benzonitril (Bunitrolol)-diphenylacetat
A: 2-Cyanophenyl
$R_1$: H
$R_2$: H
$R_3$: 1,1-Dimethylethyl
MFp.: 150 - 152° C
Ausbeute: 75,94 % der Theorie

Beispiel 19:

5-(3-((1,1-Dimethylethyl)amino)-2-hydroxypropoxy)-3,4-dihydro-1- (2H)-naphtalinon (Bunolol)-diphenylacetat
A: 5,6,7,8-Tetrahydro-5-oxo-1-naphtyl
$R_1$: H
$R_2$: H
$R_3$: 1,1-Dimethylethyl
MFp.: 170 - 172° C
Ausbeute: 75,25 % der Theorie

Beispiel 20:

1-(2-Chlor-5-methylphenoxy)-3-((1,1-dimethylethyl)amino)-2-propanol (Bupranolol)-diphenylacetat
A: 2-Chlor-5-methylphenyl
$R_1$: H
$R_2$: H
$R_3$: 1,1-Dimethylethyl
MFp.: 147 - 149° C
Ausbeute: 60,84 % der Theorie

Beispiel 21:

1-(9H-Carbazol-4-yloxy)-3-((1-methylethyl)amino)-2-propanol (Carazolol)-diphenylacetat:
A: 9 H-Carbazol-4-yl
$R_1$: H
$R_2$: H
$R_3$: 1-Methylethyl
MFp.: 148 - 150° C
Ausbeute: 62,5 % der Theorie

Beispiel 22:

1-(2-Hydroxychinolin-5-yloxy)-3-((1-methylethyl)amino)-2-propanol (Carteolol)-diphenylacetat
A: 2 Hydroxychinolin-5-yl
$R_1$: H
$R_2$: H
$R_3$: 1-Methylethyl
MFp.: 193 - 195° C
Ausbeute: 63,16 % der Theorie

Beispiel 23:

2-(2-(3-((1,1-Dimethylethyl)amino)-2-hydroxypropoxy)phenoxy)-N-methylacetamid (Cetamolol)-diphenylacetat
A: 2-((Methylcarbamoyl)-methoxy)phenyl
$R_1$: H
$R_2$: H
$R_3$: 1,1-Dimethylethyl
MFp.: 146 - 149° C
Ausbeute 92.55 % der Theorie

Beispiel 24:

N-(3-Acetyl-4-(2-hydroxy-3-((1-methylethyl)amino)propoxy)phenyl)acetamid (Diacetolol)-diphenylacetat
A: 2-Acetyl-4-acetamido-phenyl
$R_1$: H
$R_2$: H
$R_3$: 1-Methylethyl
MFp.: 143 - 145.5° C
Ausbeute: 84,74 % der Theorie

Beispiel 25:

N-(2-((3-(2-Cyanophenoxy)-2-hydroxy-propyl)amino)ethyl)-2-(4-hydroxyphenyl) acetamid (Epanolol)-diphenylacetat
A: 2-Cyanophenyl
$R_1$: H
$R_2$: H
$R_3$: 2-((4-Hydroxyphenyl)acetylamino)ethyl
MFp.: 170 - 172° C
Ausbeute: 58,8 % der Theorie

Beispiel 26:

1-((1H-Inden-4-yl)oxy)-3-((1-methylethyl)amino)-2-propanol (Indenolol)-diphenylacetat
A: (1H)-Inden-4-yl
$R_1$: H
$R_2$: H
$R_3$: 1-Methylethyl
MFp.: 129 - 130° C
Ausbeute: 86,02 % der Theorie

Beispiel 27:

4-(2-Hydroxy-3-((1-methylethyl)amino)-propoxy)-2,3,6-trimethylphenol-1-acetat(Metipranol)-diphenylacetat
A: 4-Acetoxy-2,3,6-trimethylphenyl
$R_1$: H
$R_2$: H
$R_3$: 1-Methylethyl
MFp.: 109 - 112° C
Ausbeute: 59,7 % der Theorie

Beispiel 28:

1-((1-Methylethyl)amino)-3-((2-methyl-1H-indol-4-yl)oxy)-2-propanol (Mepindolol)-diphenylacetat
A: 2-Methyl-1-H-indol-4-yl
$R_1$: H
$R_2$: H
$R_3$: 1-Methylethyl
MFp.: 162 - 165° C
Ausbeute: 91,5 % der Theorie

Beispiel 29:

1-(2-Methoxyphenoxy)-3-((1-methylethyl)amino)-2-propanol (Moprololol)-diphenylacetat
A: 2-Methoxyphenyl
$R_1$: H
$R_2$: H
$R_3$: 1-Methylethyl
MFp.: 118 - 119° C
Ausbeute: 74,1 % der Theorie

Beispiel 30:

1-(4-(2-Methoxyethyl)phenoxy)-3-((1-methylethyl)amino)-2-propanol (Metoprolol)-diphenylacetat

A: 4-(2-Methoxyethyl)phenyl
$R_1$: H
$R_2$: H
$R_3$: 1-Methylethyl
MFp.: 99 - 100° C
Ausbeute: 95,3 % der Theorie

Beispiel 31:

5-(3-((1,1-Dimethylethyl)amino)-2-hydroxypropoxy)-1,2,3,4-tetrahydro-2-3-naphtalindiol (Nadolol)-diphenylacetat
A: 5,6,7,8-Tetrahydro-6,7-dihydroxy-1-naphtyl
$R_1$: H
$R_2$: H
$R_3$: 1,1-Dimethylethyl
MFp.: 145 - 148° C
Ausbeute: 69,3 % der Theorie

Beispiel 32:

1-(-Methylenthy)amino-3(-propenyloxy)phenoxy)-2-propanol (Oxprenolol)-diphenylacetat
A: 2-(2-Propenyloxy)phenyl
$R_1$: H
$R_2$: H
$R_3$: 1-Methylethyl
MFp.: 96 - 97.5° C
Ausbeute: 94,4 % der Theorie

Beispiel 33:

N-(1-Methylethyl)-N′-(2-(4-(3-((1-methylethyl)amino)-2-hydroxypropoxy) phenyl) ethyl) harnstoff (Pafenolol)-diphenylacetat
A: 4-(2-(N′-(1-Methylethyl)ureido)ethyl)phenyl
$R_1$: H
$R_2$: H
$R_3$: 1-Methylethyl
MFp.: 85 - 89° C
Ausbeute: 97,0 % der Theorie

Beispiel 34:

N-(2-(4-(3-((1-methylethyl)amino)-2-hydroxy-propoxy)phenyl)ethyl)methyl-carbamat (Pamatolol)-diphenylacetat
A: 4-(2-((Methoxycarbonyl)amino)ethyl)phenyl
$R_1$: H
$R_2$: H
$R_3$: 1-Methylethyl
MFp.: 94 - 96° C
Ausbeute: 49,5 % der Theorie

Beispiel 35:

1-(2-Cyclopentylphenoxy)-3-((1,1-dimethylethyl)amino)-2-propanol (Penbutolol)-diphenylacetat

A: 2-Cyclopentylphenyl
$R_1$: H
$R_2$: H
$R_3$: 1,1-Dimethylethyl
MFp.: 103 - 105° C
Ausbeute: 68,6 % der Theorie

Beispiel 36:

1-(1H-Indol-4-yloxy)-3-((1-methylethyl)amino)-2-propanol (Pindolol)-diphenylacetat
A: 1H-Indol-4-yl
$R_1$: H
$R_2$: H
$R_3$: 1-Methylethyl
MFp.: 128 - 131° C
Ausbeute: 54,35 % der Theorie

Beispiel 37:

N-(4-(2-Hydroxy-3-((1-methylethyl)amino)propoxy)phenyl)acetamid (Practolol)-diphenylacetat
A: 4-Acetamido-phenyl
$R_1$: H
$R_2$: H
$R_3$: 1-Methylethyl
MFp.: 86 - 91° C
Ausbeute: 93,5 % der Theorie

Beispiel 38:

N-Cyclohexyl-N'-(4-(3-((1,1-dimethylethyl)amino)-2-hydroxypropoxy)phenyl) -harnstoff (Talinolol)-diphenylacetat
A: 4-(N'-Cyclohexylureido)phenyl
$R_1$: H
$R_2$: H
$R_3$: 1,1-Dimethylethyl
MFp.: 174 - 177° C
Ausbeute: 89,2 % der Theorie

Beispiel 39:

1-((1,1-Dimethylethyl)amino)-3-((4-morpholinyl-2,1,3-thiadiazol-5-yl)oxy)-2-propanol (Timolol)-diphenylacetat
A: 4-Morpholinyl-2,1,3-thiadiazol-5-yl
$R_1$: H
$R_2$: H
$R_3$: 1,1-Dimethylethyl
MFp.: 131 - 132° C
Ausbeute: 87,5 % der Theorie

Beispiel 40:

1-(3-Methylphenoxy)-3-((1-methylethyl)amino)-2-propanol (Toliprolol)-diphenylacetat
A: 3-Methylphenyl

$R_1$: H
$R_2$: H
$R_3$: 1-Methylethyl
MFp.: 90 - 92° C
Ausbeute: 68,96 % der Theorie

Beispiel 41:

N-(2-((3-(4-Hydroxyphenoxy)-2-hydroxy-propyl)amino)ethyl)-N′-3″-oxapentamethylenharnstoff (Xamoterol)-diphenylacetat
A: 4-Hydroxyphenyl
$R_1$: H
$R_2$: H
$R_3$: 2-(N′-(3″-oxapentamethylen)ureido)ethyl
MFp.: 140 - 142° C
Ausbeute: 86,2 % der Theorie

Beispiel 42:

1-(2,4-Dichlorphenoxy)-3-((2-(3,4-dimethoxy-phenyl)ethyl)amino)-2-propanol-diphenylacetat
A: 2,4-Dichlorphenyl
$R_1$: H
$R_2$: H
$R_3$: 2-(3,4-Dimethoxyphenyl)ethyl
MFp.: 130 - 132° C
Ausbeute: 91,13 % der Theorie

Beispiel 43:

4-(2-Hydroxy-3-((1-methyl-3-phenylpropyl)amino)propoxy)phenylacetamid-diphenylacetat
A: 4-Carbamoylmethyl-phenyl
$R_1$: H
$R_2$: H
$R_3$: 1-Methyl-3-phenylpropyl
MFp.: 137 - 140° C
Ausbeute: 83,85 % der Theorie

Beispiel 44:

4-(3-((1,1-Dimethylethyl)amino)-2-pivaloyloxy-propoxy)-9-fluorenon-diphenylacetat
A: 9-Fluorenon-4-yl
$R_1$: Pivaloyl
$R_2$: H
$R_3$: 1,1-Dimethylethyl
MFp.: 127 - 128,5° C
Ausbeute: 82,9 % der Theorie

Beispiel 45:

N-(2-((3-(2-Cyanophenoxy)-2-hydroxy-propyl)amino)ethyl)N′-phenylharnstoff-diphenylacetat
A: 2-Cyanophenyl
$R_1$: H
$R_2$: H

$R_3$: 2-(N'-phenylureido)ethyl
MFp.: 140 - 143° C
Ausbeute: 88,33 % der Theorie

Beispiel 46:

1-((5-Hydroxy-napht-1-yloxy)-3-((1-methylethyl)amino)-2-propanol
A: 5-Hydroxy-1-naphtyl
$R_1$: H
$R_2$: H
$R_3$: 1-Methylethyl
MFp.: 169 - 174° C
Ausbeute: 86,46 % der Theorie

Beispiel 47:

1-(4-((2-Cyclopropylmethoxy)ethyl)phenoxy)-3-((1-methylethyl)amino)2-propanol (Betaxolol)
A: 4-((2-Cyclopropylmethoxy)ethyl)phenyl
$R_1$: H
$R_2$: H
$R_3$: 1-Methylethyl
MFp.: 68 - 70° C
Ausbeute: 78,8 % der Theorie

Beispiel 48: Eintopfverfahren

100 g N'-(3-Acetyl-4-hydroxy)phenyl-N,N-diethylharnstoff werden mit 80 ml Dimethylsulfoxid und 170 ml Epibromhydrin versetzt, unter Argonatmosphäre 26,9 g 91,5 % Kalilauge zugegeben und für 4 Stunden bei 30 - 32° C gerührt. Nach Beendigung der Umsetzung werden 120 ml $H_2O$ zugesetzt und das überschüssige Epibromhydrin in Vakuum azeotrop abdestilliert.
Der Rückstand wird abgekühlt, mit 83,7 ml (1,1-Dimethylethyl)amin (0,8 Mol) und 83,7 ml $H_2O$ versetzt und über Nacht bei 20 bis 25° C gerührt. Das Reaktionsgemisch wird mit 50 ml Aceton sowie 20 ml $H_2O$ versetzt und angeimpft. Nach der beginnenden Kristallisation werden noch 230 ml $H_2O$ zugetropft und 5 Stunden auf 5° C abgekühlt. Das ausgefallene Kristallisat wird abgesaugt, mit $H_2O$ gewaschen und an der Luft getrocknet. Der Rückstand der Rohbase (145,4 g N'-(3-Acetyl-4-(3-((1,1-dimethylethyl)amino)-2-hydroxypropoxy)phenyl)-N,N-diethylharnstoff-hydrat) wird bei 27° C in 850 ml Aceton gelöst, klar filtriert und mit einer Lösung von 77,3 g Diphenylessigsäure in 250 ml Aceton versetzt und das gebildete Diphenylacetat zunächst ohne Kühlung, sodann 4 Stunden bei 0° C zur Kristallisation gebracht.

Ausbeute an N'-3-Acetyl-4-(3-((1,1-dimethylethyl)amino)-2-hydroxypropoxy) phenyl)-N,N-diethylharnstoff (Celiprolol)-diphenylacetat 192,65 g = 89.4 % der Theorie, bezogen auf die 145,4 g als Rohbase erhaltenen N-(3-Acetyl-4-(3-((1,1-dimethylethyl)amino)-2-hydroxypropoxy)phenyl)-N,N-diethylharnstoff-hydrat s.o. mit einem Wassergehalt von 4,66 Gew.%
MFp.: 140 - 142° C
Das so erhaltene Diphenylacetat ist analysenrein und zeigt im Dünnschichtchromatogramm in mehreren Laufmitteln einen einheitlichen Spot.

Beispiel 49:

N'-(3-Acetyl-4-(3-((1,1-dimethylethyl)amino)-2-hydroxypropoxy)phenyl)- N,N-diethylharnstoff (Celiprolol)
5,9 g des nach einem der Beispiele 1 - 6 erhaltenen Diphenylacetats werden in 50 ml Chloroform und 10 ml 5%iger Natronlauge gerührt, die Chloroformphase abgetrennt, über $Na_2SO_4$ getrocknet und im Vakuum eingedampft, und der kristalline Rückstand mit wenig Aceton in der Kälte digeriert und abgesaugt. Man erhält so 3,45 g dünnschichtchromatographisch reines Produkt in Form farbloser Kristalle vom Schmelzpunkt 116 - 118° C.

Beispiel 50:

N'-(3-Acetyl-4-(3-(1,1-dimethylethyl)amino)-2-hydroxypropoxy)phenyl)- N,N-dimethylharnstoff (Celiprolol)-hydrochlorid.
625 g eines nach einem der Beispiele 1 - 6 erhaltenen Diphenylacetats werden in 4700ml Aceton suspendiert, 92,5 ml Wasser zugegeben und 15 Minuten bei Raumtemperatur gerührt.
Sodann werden 92.5 ml konzentrierte HCl (41.7 g HCl in 100 ml) unter Rühren zugesetzt und zunächst 30 Minuten bei Raumtemperatur und dann bei +2° C zur Vervollständigung der Kristallisation stehen gelassen.
Das entstandene Kristallisat wird abgesaugt, mit Aceton gewaschen, nochmals in Aceton suspendiert, 1 Stunde gerührt, neuerlich abgesaugt, nachgewaschen und an der Luft getrocknet.
Man erhält so 422.15 g (96.1 % der Theorie) dünnschichtchromatographisch reines Hydrochlorid vom MFp. 198 - 200° C

Rückgewinnung der Diphenylessigsäure:

Die acetonische Mutterlauge wird im Vakuum auf ca. 400 ml eingeengt, mit 1200 ml Wasser versetzt, 20 ml HCl konz. zugegeben, die ausgefallene Diphenylessigsäure abgesaugt, mit Wasser gewaschen und getrocknet. Die auf diese Weise zurückgewonnene Diphenylessigsäure kann ohne weitere Reinigung wieder für die Herstellung eines Diphenylacetats eingesetzt werden.
Ausbeute: 219,0 g = 97,7 % der Theorie.

Beispiel 51:

1-((1-Methylethyl)amino)-3-(1-naphtyloxy)-2-propanol (Propranolol)-hydrochlorid 9.3 g eines nach Beispiel 7 erhaltenen Diphenylacetats werden in 93 ml Aceton suspendiert, 2,48 ml alkoholische HCl (29 g HCl in 100 ml) zugegeben und das ausgeschiedene Hydrochlorid nach 1,5 Stunden abgesaugt und mit Aceton gewaschen.
Ausbeute 5.57 g (95.5 % der Theorie)
MFp.: 163 - 165° C
Das Hydrochlorid zeigt im Dünnschichtchromatogramm in mehreren Systemen einen einheitlichen Spot.
Stationäre Phase: Kieselgel $HF_{60}$
Mobile Phase: Benzol/Aceton/Eisessig/Wasser; 40/50/30/20; v/v/v/v
Entwicklung: UV oder Besprühen mit Jod-Lösung

Nach der in den Beispielen 47 - 49 beschriebenen Arbeitsmethode wurden aus den entsprechenden Diphenylacetaten noch folgende Aryloxy-propanolamine oder deren pharmazeutisch verträgliche Salze hergestellt:

| | |
|---|---|
| Enciprazin-dihydrochlorid: | MFp.: 196 - 197° C |
| Cloranolol-hydrochlorid: | MFp.: 210 - 212° C |
| Xibenolol: | MFp.: 71 - 72° C |
| Acebutolol-hydrochlorid: | MFp.: 141 - 143° C |
| Alprenolol-hydrochlorid: | MFp.: 107 - 109° C |
| Atenolol: | MFp.: 146 - 148° C |
| Bevantolol-hydrochlorid: | MFp.: 137 - 138° C |
| Bucindolol-hydrochlorid: | MFp.: 185 - 187° C |
| Bufetolol-hydrochlorid: | MFp.: 153 - 157° C |
| Bunitrolol-hydrochlorid: | MFp.: 163 - 165° C |
| Bunolol-hydrochlorid: | MFp.: 225 - 227° C |
| Bupranolol-hydrochlorid: | MFp.: 220 - 222° C |
| Carazolol-hydrochlorid: | MFp.: 234 - 235° C |
| Carteolol-hydrochlorid: | MFp.: 277 - 278° C |
| Cetamolol-hydrochlorid: | MFp.: 145 - 147° C |
| Diacetolol: | MFp.: 127 - 130° C |
| Indenolol-hydrochlorid: | MFp.: 147 - 148° C |
| Metipranol: | MFp.: 105 - 107° C |
| Mepindolol: | MFp.: 100 - 102° C |
| Moprolol-hydrochlorid: | MFp.: 110 - 112° C |
| Metoprolol-hydrochlorid: | MFp.: 83° C |
| Oxprenolol: | MFp.: 78 - 80° C |
| Pafenolol | |
| Pamatolol-hydrochlorid: | MFp.: 106° C |
| Penbutolol-hydrochlorid: | MFp.: 123 - 124° C |
| Pindolol: | MFp.: 171 - 178° C |
| Practolol: | MFp.: 134 - 136° C |
| Talinolol: | MFp.: 142 - 144° C |
| Timolol-hydrochlorid: | MFp.: 161 - 163° C |
| Xamoterol-hemifumarat: | MFp.: 168 - 169° C |

1-(2,4-Dichlorphenoxy)-3-((2-(3,4-dimethoxy-phenyl)-ethyl)-amino)-2- propanolhydrochlorid: Fp = 151 - 152° C

4-(2-Hydroxy-3-((1-methyl-3-phenylpropyl)amino)propoxy)-phenylacetamid: Fp = 106 - 108° C

4-(3-(1,1-Dimethylethyl)amino-2-pivaloyloxy-propoxy)-9-fluorenon-hydrochlorid: Fp = 228 - 231° C

N-(2-((3-(2-Cyanophenoxy)-2-hydroxy-propyl)-amino)-ethyl>-N'-phenylharnstoff Fp = 144 - 145° C

1-(4-(2-(Cyclopropylmethoxy)-ethyl)-phenoxy)-3-((1-methylethyl)-amino)-2-propanolhydrochlorid: Fp = 116° C

**Ansprüche**

1. Salze von Aryloxy-propanolaminen mit Diphenylessigsäure der allgemeinen Formel I

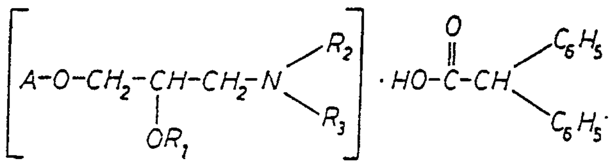 (I)

in der
A
a) einen substituierten Phenylrest der allgemeinen Formel II

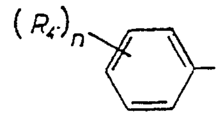 (II)

worin
n für eine ganze Zahl von 1 bis 4 steht, die Substituenten $R_4$ entweder gleich oder verschieden sind und unabhängig voneinander für Niederalkyl, C-2- oder C-3-Niederalkenyl, $C_5$ oder $C_6$-Cycloalkyl, Niederalkoxy, C-2-oder C-3-Niederalkenoxy, Cycloalkylalkoxy, Tetrahydrofuranyl-methoxy, Niederalkoxyniederalkyl, Niederalkanoyl, Halogen, Hydroxy, Cyano, Carbonamido, Acyloxy, Reste der Formel III oder IV

$$-O-CH_2-X \quad (III) \quad \text{oder} \quad -(CH_2)_m-X \quad (IV)$$

stehen, wobei
X in den Formeln III und IV wiederum Carbamoyl, Alkylcarbamoyl, Dialkylcarbamoyl, Ureido, N'-Alkylureido, N'-Cycloalkylureido, N,N'-Dialkylureido, N',N'-Dialkylureido oder Alkoxycarbonylamino und m in der Formel IV Null oder eine ganze Zahl von 1 bis 3 darstellen
b) den 1-Naphtyl-, 2-Naphtyl-, 6,7-Dihydroxy-1-naphtyl-, 5-Hydroxy-1-naphtyl-, 5,6,7,8-Tetrahydro-6,7-dihydroxy-1-naphtyl-, 5,6,7,8-Tetrahydro-5-oxo-1-naphtyl, 9-Fluorenon-4-yl oder Inden-4-ylrest oder
c) einen Indolyl-, Methylindolyl, 2-Hydroxy-chinolin-, Carbazolyl- oder 4-Morpholinyl-2,1,3-thiadiazolylrest bedeutet,
$R_1$ Wasserstoff oder einen geradkettigen oder verzweigten
$C_2$ bis $C_5$-Alkanoyl- oder Aroylrest,
$R_2$ Wasserstoff und
$R_3$ einen verzweigten $C_3$ bis $C_6$-Alkylrest, einen unsubstituierten oder durch Hydroxy oder Alkoxy substituierten, geradkettigen oder verzweigten Phenylniederalkyl oder Indolylniederalkylrest, einen Rest der formel V

$$-(CH_2)_1-Y \quad (V),$$

wobei in der Formel V 1 für eine ganze Zahl 1 oder 2 und Y für einen N'-Phenylureido, N'-Pentamethylenureido N'-3"-Oxapentamethylenureido- oder einen unsubstituierten oder substituierten Phenylacetyl-aminorest steht, oder $R_2$ und $R_3$ unter sich verbunden und zusammen mit dem anliegenden Stickstoffatom einen unsubstituierten oder substituierten 5-oder 6glied rigen gesättigten heterocyclischen Rest bedeuten.

2. Salze nach Anspruch 1, von N'(3-Acetyl-4(3-((1,1-dimethylethyl)ami-no)-2-hydroxypropoxy)phenyl)-N,N-diethylharnstoff (Celiprolol), 1-((1-Methylethyl)amino)-3-(1-naphtyloxy)-2-propanol (Propranolol), 1-(2,3-Dimethylphenoxy)-3-(1,1-dimethylethyl)amino-2-propanol (Xibenol), 1-(4-(2-Methoxyphenyl)-piperazin-1-yl)-3-((3,4,5-trimethoxy)phenoxy)-2-propanol (Enciprazin), 1-(2,5-Dichlorphenoxy)-3-((1,1-dimethylethyl)-amino)-2-propanol (Cloranolol), 1-(2-Chlor-5--methylphenoxy)-3-((1,1-dimethylethyl) amino)-2-propanol (Bupranolol>, 1-(4-(2-Methoxyethyl)phenoxy)-3-((1-methylethyl) amino)-2-propanol (Metoprolol), 4-(2-Hydroxy-3-((1-methylethyl)amino)propoxy) phenylacetamid (Atenolol), N-(3-Acetyl-4-(2-hydroxy-3-((1-methylethyl) amino)propoxy)phenyl)acetamid (Diacetolol), 1-((1-Methylethyl) amino)3-(2-(2-propenyloxy)phenoxy)-2-propanol (Oxprenolol), 1-((1,1-Dimethylethyl)amino)-3-(2-((tetrahydro-2-furanyl)-methoxy)phenoxy)-2-propanol (Bufetolol), 1-(3-Methylphenoxy)-3-((2-3,4-dimethoxyphenyl)ethyl)amino)-2-propanol (Bevantolol), 2-(3-((1,1-Dimethylethyl)amino)-2-hydroxypropoxy) benzonitril (Bunitrolol), N-(3-Acetyl-4-(2-hydroxy-3-((1-methylethyl) amino)propoxy)phenyl)butyramid (Acebutolol), 1-((1,1-Dimethylethyl) amino)-3-((4-(morpholinyl)-2,1,3-thiadiazol-5-yl)-2-propanol (Timolol), N-(2-((3-(2-Cyanophenoxy)-2-hydroxy-propyl)amino)ethyl)-2-(4-hydroxy-phenyl) acetamid (Epanolol), 2-(2-Hydroxy-3-((2-(1H)-indol-3-yl-1,1-dimethylethyl)amino)-propoxy)benzonitril (Bucindolol), 1-(2,4-Dichlor-phenoxy)-3-(3,4-dimethoxy-beta-phenylethylamino)-2-propanol, 4-(2-Hydroxy-3-((1-methyl-3-phenylpropyl)-amino)-propoxy)phenyl-acetamid, N-(2-((3-(2-Cyanophenoxy)-2-hydroxy-propyl)amino)ethyl)-N` -phenylharn-'stoff, 5-(3((1,1Dimethylethyl) amino)-2-hydroxypropoxy)-3,4-dihydro-1-(2H)-naphtalinon (Bunolol), 2-(3-((1,1-Dimethylpropyl)amino)-2-hydroxy-propoxy)-benzonitril (Penirolol)

3. Verfahren zur Herstellung von Salzen nach den Ansprüchen 1 oder 2, dadurch gekennzeichnet, daß man ein Aryloxy-propanolamin der allgemeinen Formel VI

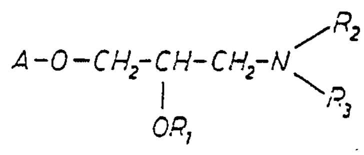

(VI)

in der

A, $R_1$, $R_2$ und $R_3$ die unter Formel 1 angegebene Bedeutung haben, in einem gegenüber den Reaktionspartnern inerten Lösungsmittel oder Lösungsmittelgemisch bei Temperaturen von Raumtemperatur bis zur Siedetemperatur des Lösungsmittels oder der niedrigstsiedenden Lösungsmittelkomponente mit einer mindestens stöchiometrischen Menge an Diphenylessigsäure umsetzt, das sich bei Raumtemperatur oder bevorzugt in der Kälte abscheidende Diphenylacetat der Formel 1 aus dem bei der Umsetzung erhaltenen Reaktionsgemisch abtrennt und gegebenenfalls aus einem organischen Lösungsmittel umkristallisiert.

4. Verfahren nach Anspruch 3, dadurch gekennzeichnet, daß man als Aryloxy-propanolamin der Formel VI ein Rohprodukt einsetzt, wie es auf einem für die Herstellung von Aryloxy-propanolaminen üblichen Syntheseweg nach der Abtrennung aus dem Reaktionsgemisch ohne weiteren Reinigungsschritt erhalten wird.

5. Verfahren nach Anspruch 4, dadurch gekennzeichnet, daß man als Aryloxypropanolamin der Formel VI ein Rohprodukt ein-setzt, wie es durch Umsetzung eines 1-Aryloxy-2,3-epoxy-propans der allgemeinen Formel VII

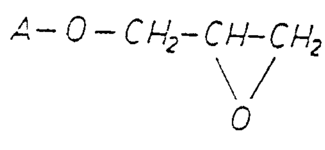

(VII)

oder einer Verbindung der allgemeinen Formel VIII

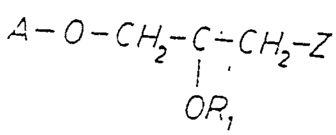

(VIII)

mit der äquivalenten Menge oder einem geringen, beispielsweise zwei- bis dreifachen, Überschuß eines

Amins der allgemeinen Formel IX

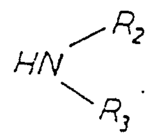 (IX),

wobei in den obigen Formeln A, $R_1$, $R_2$ und $R_3$ die unter Formel I angegebene Bedeutung haben und Z für eine leicht abspaltbare Abgangsgruppe steht, nach der Abtrennung aus dem Reaktionsgemisch erhalten wird.

6. Verwendung der Salze der allgemeinen Formel I nach den Ansprüchen 1 oder 2 zur Herstellung von chemisch reinen Aryloxy-propanolaminen der allgemeinen Formel VI oder von deren pharmazeutisch verträglichen Säureadditionssalzen, indem man aus dem Diphenylacetat der allgemeinen Formel I das Aryloxy-propanolamin der Formel VI durch Zusatz von Basen wiedergewinnt und gewünschtenfalls mit einer Säure in ein pharmazeutisch verträgliches Additionssalz überführt.

7. Verwendung nach Anspruch 6 zur Herstellung von pharmazeutisch verträglichen Säureadditionssalzen von Aryloxy-propanolaminen der allgemeinen Formel VI, indem man das Diphenylacetat der allgemeinen Formel I mit einer geeigneten Säure umsalzt.

Patentansprüche für folgende Vertragsstaaten AT,ES,GR:

1. Verfahren zur Herstellung von neuen Salzen von Aryloxy-propanolaminen mit Diphenylessigsäure der allgemeinen Formel I

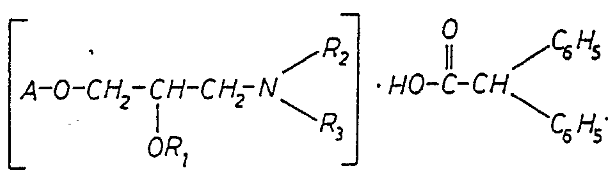 (I)

in der

A

a) einen substituierten Phenylrest der allgemeinen Formel II

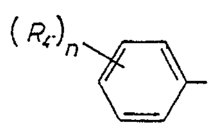 (II)

worin n für eine ganze Zahl von 1 bis 4 steht, die Substituenten $R_4$ entweder gleich oder verschieden sind und unabhängig voneinander für Niederalkyl, C-2- oder C-3-Niederalkenyl, $C_5$ oder $C_6$-Cycloalkyl, Niederalkoxy, C-2-oder C-3-Niederalkenoxy, Cycloalkylalkoxy, Tetrahydrofuranyl-methoxy, Niederalkoxyniederalkyl, Niederalkanoyl, Halogen, Hydroxy, Cyano, Carbonamido, Acyloxy, Reste der Formel III oder IV

$$-O-CH_2-X \quad (III) \quad oder \quad -(CH_2)_m-X \quad (IV),$$

stehen, wobei

X in den Formeln III und IV wiederum Carbamoyl, Alkylcarbamoyl, Dialkylcarbamoyl, Ureido, N'-Alkylureido, N'-Cycloalkylureido, N,N'-Dialkylureido, N',N'-Dialkylureido oder Alkoxycarbonylamino und m in der Formel IV Null oder eine ganze Zahl von 1 bis 3 darstellen

b) den 1-Naphtyl-, 2-Naphtyl-, 6,7-Dihydroxy-1-naphtyl-, 5-Hydroxy-1-naphtyl-, 5,6,7,8-Tetrahydro-6,7-dihydroxy-1-naphtyl-, 5,6,7,8-Tetrahydro-5-oxo-1-naphtyl, 9-Fluorenon-4-yl oder Inden-4-ylrest oder
c) einen Indolyl-, Methylindolyl, 2-Hydroxy-chinolin-, Carbazolyl- oder 4-Morpholinyl-2,1,3-thiadiazolylrest bedeutet,
$R_1$ Wasserstoff oder einen geradkettigen oder verzweigten
$C_2$ bis $C_5$-Alkanoyl- oder Aroylrest,
$R_2$ Wasserstoff und
$R_3$ einen verzweigten $C_3$ bis $C_6$-Alkylrest, einen unsubstituierten oder durch Hydroxy oder Alkoxy substituierten, geradkettigen oder verzweigten Phenylniederalkyl oder Indolylniederalkylrest, einen Rest der formel V

$$-(CH_2)_1-Y \qquad (V),$$

wobei in der Formel V 1 für eine ganze Zahl 1 oder 2 und Y für einen N'-Phenylureido, N'-PentamethylenureidoN'-3"-Oxapentamethylenureido-oder einen unsubstituierten oder substituierten Phenylacetyl-aminorest steht, oder $R_2$ und $R_3$ unter sich verbunden und zusammen mit dem anliegenden Stickstoffatom einen unsubstituierten oder substituierten 5-oder 6glied-rigen gesättigten heterocyclischen Rest bedeuten, dadurch gekennzeichnet, daß man ein Aryloxy-propanolamin der allgemeinen Formel VI

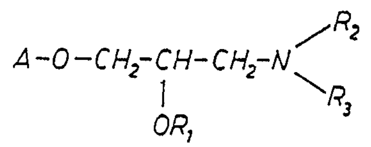

(VI)

in der
A, $R_1$, $R_2$ und $R_3$ die unter Formel I angegebene Bedeutung haben, in einem gegenüber den Reaktionspartnern inerten Lösungsmittel oder Lösungsmittelgemisch bei Temperaturen von Raumtemperatur bis zur Siedetemperatur des Lösungsmittels oder der niedrigstsiedenden Lösungsmittelkomponente mit einer mindestens stöchiometrischen Menge an Diphenylessigsäure umsetzt, das sich bei Raumtemperatur oder bevorzugt in der Kälte abscheidende Diphenylacetat der Formel I aus dem bei der Umsetzung erhaltenen Reaktionsgemisch abtrennt und gegebenenfalls aus einem organischen Lösungsmittel umkristallisiert.

**2.** Verfahren nach Anspruch 1, dadurch gekennzeichnet, daß man N'-(3-Acetyl-4-(3-((1,1-dimethylethyl)-amino)-2-hydroxypropoxy)phenyl)-N, N diethylharnstoff (Celiprolol), 1-((1-Methylethyl)amino)-3-(1-naphtyloxy)-2-propanol (Propranolol), 1-(2,3-Dimethylphenoxy)-3-1,1-dimethylethyl)amino-2-propanol (Xibenol), 1-(4-(2-Methoxhenyl)-piperazin-1-yl)-3-((3,4,5-trimethoxy)phenoxy)-2-propanol (Enciprazin), 1-(2,5-Dichlorphenoxy)-3-((1,1-dimethylethyl)amino)-2-propanol (Cloranolol), 1-(2-Chlor-5-methylphenoxy)-3-((1,1-dimethylethyl) amino)-2-propanol (Bupranolol), 1-(4-(2-Methoxyethyl)phenoxy)-3-((1-methylethyl) amino)-2-propanol (Metoprolol), 4-(2-Hydroxy-3-((1-methylethyl)amino)propoxy) phenylacetamid (Atenolol), N-(3-Acetyl-4-(2-hydroxy-3-((1-methylethyl) amino)propoxy)Phenyl)acetamid (Diacetolol), 1-(-(1-Methylethyl) amino)-3-(2-(2-propenyloxy)phenoxy-2-propanol (Oxprenolol), 1-((1,1-Dimethylethyl)-amino)-3-(2-((tetrahydro-2-furanyl)methoxy)phenoxy)-2-propanol (Bufetolol), 1-(3-Methylphenoxy)-3-((2-(3,4-dimethoxyphenyl) ethyl)amino)-2-propanol (Bevantolol), 2-(3-((1,1-Dimethylethyl)amino)-2-hydroxypropoxy) benzonitril (Bunitrolol), N-(3-Acetyl-4-(2-hydroxy-3-((1-methylethyl) amino)propoxy)phenyl)-butyramid (Acebutolol), 1-((1,1-Dimethylethyl) amino)-3-((4-(morpholinyl)-2,1,3-thiadiazol-5-yl)-2-propanol (Timolol), N-(2-((3-(2-Cyanophenoxy)-2-hydroxy-propyl)amino)ethyl)-2-(4-hydroxy-phenyl)acetamid (Epanolol), 2-(2-Hydroxy-3-((2-(1H)indol-3-yl-1,1-dimethylethyl)amino)-propoxy)benzonitril (Bucindolol), 1-(2,4-Dichlor-phenoxy)-3-(3,4-dimethoxy-beta-phenylethylamino)-2-propanol, 4-(2-Hydroxy-3-((1-methyl-3-phenylpropyl)amino)-propoxy)phenyl-acetamid, N-(2-((3-(2-Cyanophenoxy)-2-hydroxy-propyl)-amino)ethyl)-N'-phenylharnstoff, 5-(3-((1,1-Dimethyl) amino)-2-hydroxypropoxy)-3,4-dihydro-1-(2H)-naphtalinon (Bunolol), oder 2-(3-((1,1-Dimethylpropyl)amino)-2-hydroxy-propoxy)-benzonitril (Penirol) mit Diphenylessigsäure umsetzt.

**3.** Verfahren nach Anspruch 1 oder 2, dadurch gekennzeichnet, daß man als Aryloxy-propanolamin der

Formel VI ein Rohprodukt einsetzt, wie es auf einem für die Herstellung von Aryloxy-propanolaminen üblichen Syntheseweg nach der Abtrennung aus dem Reaktionsgemisch ohne weiteren Reinigungsschritt erhalten wird.

**4.** Verfahren nach einem der Ansprüche 1 bis 3, dadurch gekennzeichnet, daß man als Aryloxypropanolamin der Formel VI ein Rohprodukt einsetzt, wie es durch Umsetzung eines 1-Aryloxy-2,3-epoxypropans der allgemeinen Formel VII

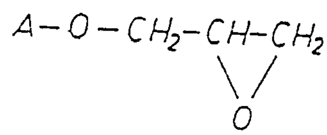

(VII)

oder einer Verbindung der allgemeinen Formel VIII

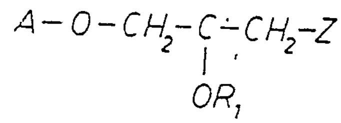

(VIII)

mit der äquivalenten Menge oder einem geringen, beispielsweise zwei- bis dreifachen, Überschuß eines Amins der allgemeinen Formel IV

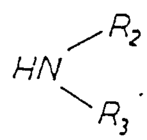

(IX),

wobei in den obigen Formel A, $R_1$, $R_2$ und $R_3$ die unter Formel I angegebene Bedeutung haben und Z für eine leicht abspaltbare Abgangsgruppe steht, nach der Abtrennung aus dem Reaktionsgemisoh erhalten wird.

**5.** Verwendung eines nach einem der Ansprüche 1 - 4 hergestellten Salzes der allgemeinen Formel 1 zur Herstellung von chemisch reinen Aryloxy-propanolaminen der allgemeinen Formel VI oder von deren pharmazeutisch verträglichen Säureadditionssalzen, indem man aus dem Diphenylacetat der allgemeinen Formel 1 das Aryloxy-propanolamin der Formel VI durch Zusatz von Basen wiedergewinnt und gewünschtenfalls mit einer Säure in ein pharmazeutisch verträgliches Additionssalz überführt.

**6.** Verwendung eines nach einem der Ansprüche 1 bis 4 hergestellten Salzes der allgemeinen Formel 1 zur Herstellung von pharmazeutisch verträglichen Säureadditionssalzen von Aryloxy-propanolaminen der allgemeinen Formel VI, indem man das Diphenylacetat der allgemeinen Formel 1 mit einer geeigneten Säure umsalzt.

## Claims

**1.** Salts of aryloxypropanolamines with diphenylacetic acid of the general formula I

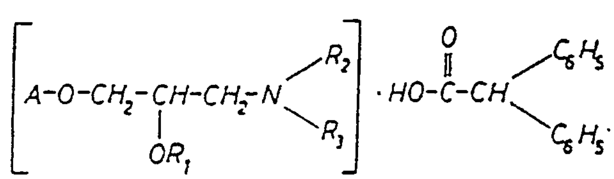

(I)

in which

A denotes

a) a substituted phenyl radical of the general formula II

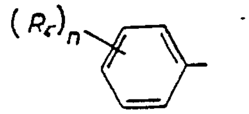

(II)

wherein
n represents an integer from 1 to 4 and the substituents $R_4$ are either identical or different and independently of one another represent lower alkyl, C-2- or C-3-lower alkenyl, $C_5$- or $C_6$-cycloall, lower alkoxy, C-2- or C-3-lower alkenyloxy, cycloalkylalkoxy, tetrahydrofuranylmethoxy, lower alkoxy-lower alkyl, lower alkanoyl, halogen, hydroxyl, cyano, carboxamido, acyloxy or radicals of the formula III or IV

$$-O-CH_2-X \quad (III) \qquad \text{or} \qquad -(CH_2)_m-X \quad (IV)$$

in which
x in formulae III and IV in turn represents carbamoyl, alkylcarbamoyl, dialkylcarbamoyl, ureido, N'-alkylureido, N'-cycloalkylureido, N,N'dialkylureido, N' ,N' -dialkylureido or alkoxycarbonylamino and m in formula IV represents zero or an integer from 1 to 3,
b) the 1-naphthyl, 2-naphthyl, 6,7-dihydroxy-1-naphthyl, 5-hydroxy-1-naphthyl, 5,6,7,8-tetrahydro-6,7-dihydroxy-1-naphthyl, 5,6,7,8-tetrahydro-5-oxo-1-naphthyl, 9-fluorenon-4-yl or inden-4-yl radical or
c) an indolyl, methylindolyl, 2-hydroxyquinolyl, carbazolyl or 4-morpholinyl-2,1,3-thiadiazolyl radical, $R_1$ denotes hydrogen or a straight-chain or branched $C_2$- to $C_5$-alkanoyl or aroyl radical, $R_2$ denotes hydrogen and $R_3$ denotes a branched $C_3$- to $C_6$-alkyl radical, an unsubstituted or hydroxyl-substituted or alkoxysubstituted straight-chain or branched phenyl-lower alkyl or indolyl-lower alkyl radical, a radical of the formula V

$$-(CH_2)_1-Y \qquad (V)$$

where, in formula V, 1 represents the integer 1 or 2 and Y represents an N'-phenylureido, N'-pentamethyleneureido or N'-3''-oxapentamethylene-ureido radical or an unsubstituted or substituted phenylacetylamino radical, or $R_2$ and $R_3$, bonded to one another and together with the adjacent nitrogen atom, denote an unsubstituted or substituted 5-membered or 6-membered saturated heterocyclic radical.

**2.** Salts according to Claim 1, of N'-(3-acetyl-4-(3-((1, 1-dimethylethyl) -amino)-2-hydroxypropoxy)-phenyl)-N,N-diethylurea (celiprolol), 1-((1methylethyl)-amino) -3-( 1-naphthyloxy) -2-propanol (propranolol), 1-(2,3-dimethylphenoxy)-3-(1,1-dimethylethyl)-amino-2-propanol (xibenol), 1-(4-(2-methoxyphenyl)-piperazin-1-yl)-3-((3,4,5-trimethoxy)-phenoxy) -2-propanol (enciprazine), 1- (2,5-dichlorophenoxy) -3-(-(1,1-dimethylethyl)-amino)-2-propanol (cloranolol), 1-(2-chloro-5-methylphenoxy)-3-((1, 1-dimethylethyl)-amino)-2-propanol (bupranolol), 1-(4-( 2-methoxyethyl ) -phenoxy) -3- ((1-methylethyl)-amino) - 2-propanol (metoprolol), 4-(2-hydroxy-3-((1-methylethyl) -amino) -propoxy) -phenyl) -acetamide (atenolol), N-(3-acetyl-4-(2-hydroxy-3-((1-methylethyl) -amino) -propoxy) -phenyl) -acetamide (diacetolol), 1((1-methylethyl)-amino)-3-(2-(2-propenyloxy)-phenoxy)-2-propanol (oxprenolol), 1-((1,1-dimethylethyl)-amino) -3-(2-((tetrahydro-2-furanyl)-methoxy)-phenoxy)-2-propanol (bufetolol), l-(3-methylphenoxy)-3-((2-(3,4-dimethoxyphenyl)-ethyl)-amino)-2-propanol (bevantolol), 2-(3-((1,1-dimethylethyl)-amino)-2-hydroxypropoxy)-benzonitrile (bunitrolol), N-(3-acetyl-4-(2-hydroxy-3-((1-methylethyl) -amino) -propoxy) -phenyl) -butyramide (acetbutolol), 1-((1,1-dimethylethyl)-amino)-3-((4-(morpholinyl) -2,1, 3-thiadiazol-5-yl) -2-propanol (timolol), N(2-((3-(2-cyanophenoxy)-2-hydroxypropyl)-amino)-ethyl)-2-(4-hydroxyphenyl)-acetamide (epanolol), 2-(2-hydroxy-3-((2(1H)-indol-3-y1-1,1-dimethylethyl)-amino)-propoxy)-benzonitrile (bucindolol), 1-(2,4-dichlorophenoxy)-3-(3,4-dimethoxy-beta-phenylethyl-amino)-propanol, 4-(2-hydroxy-3-((l-methyl-3-phenylpropyl)-amino)-propoxy)-phenylacetamide, N-(2-((3-(2-cyanophenoxy)-2-hydroxypropyl)-amino)-ethyl)-N'-phenylurea, 5-(3-((1,1-dimethylethyl)-amino)-2-hydroxypropoxy) -3 ,4-dihydro-1(2H) -naphthalenone (bunolol) and 2-(3-((1-dimethylpropyl)-amino)-2-hydroxypropoxy)-benzonitrile

(penirolol).

3. Process for the preparation of salts according to Claim 1 or 2, characterized in that an aryloxypropanolamine of the general formula VI

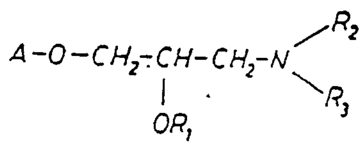

(VI)

in which
A, $R_1$, $R_2$ and $R_3$ have the meaning stated under formula I, is reacted with at least the stoichiometric amount of diphenylacetic acid in a solvent or solvent mixture which is inert to the reactants, at temperatures from room temperature to the boiling point of the solvent or of the lowest-boiling solvent component, the diphenyl acetate of the formula I which is precipitated at room temperature or preferably at low temperature is separated off from the reaction mixture obtained in the reaction and is optionally recrystallized from an organic solvent.

4. Process according to Claim 3, characterized in that a crude product as obtained by a synthesis customary for the preparation of aryloxypropanolamines, after separation from the reaction mixture, without a further purification step, is used as the aryloyypropanolamine of the formula VI.

5. Process according to Claim 4, characterized in that a crude product as obtained by reacting a 1-aryloxy-2,3-epoxypropane of the general formula VII

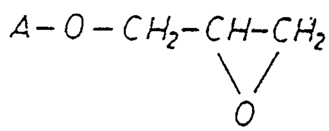

(VII)

or a compound of the general formula VIII

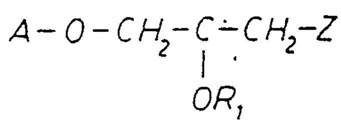

(VIII)

with the equivalent amount or a small excess, for example a two-fold to three-fold excess, of an amine of the general formula IX

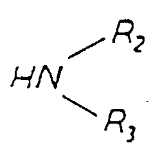

(IX)

where, in the above formulae A, $R_1$, $R_2$ and $R_3$ have the meaning stated under formula I and Z represents a leaving group which can be readily eliminated, after separation from the reaction mixture, is used as the aryloxypropanolamine of the formula VI.

6. Use of the salts of the general formula I according to Claim 1 or 2 for the preparation of chemically pure aryloxypropanolamines of the general formula VI or of their pharmaceutically tolerated acid addition salts, by recovering the aryloxypropanolamine of the formula VI from the diphenyl acetate of the general formula I by the addition of bases, and, if desired, converting it with an acid into a pharmaceutically tolerated addition salt.

7. Use according to Claim 6 for the preparation of pharmaceutically tolerated acid addition salts of aryloxypropanolamines of the general formula VI, by reacting the diphenyl acetate of the general

formula I with a suitable acid to form another salt.

Claims for the following contracting states: AT, ES, GR

1. Process for the preparation of salts of aryloxypropanolamines with diphenylacetic acid of the general formula I

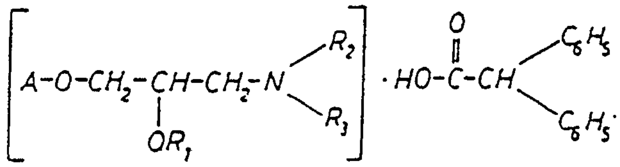

(I)

in which
A denotes
a) a substituted phenyl radical of the general formula II

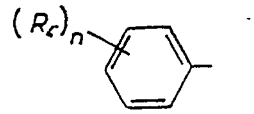

(II)

wherein
n represents an integer from 1 to 4 and the substituents $R_4$ are either identical or different and independently of one another represent lower alkyl, C-2- or C-3-lower alkenyl, $C_5$- or $C_6$-cycloalkyl, lower alkoxy, C-2- or C-3-lower alkenyloxy, cycloalkylalkoxy, tetrahydrofuranylmethoxy, lower alkoxy-lower alkyl, lower alkanoyl, halogen, hydroxyl, cyano, carboxamido, acyloxy or radicals of the formula III or IV

$$-O-CH_2-X \quad (III) \qquad \text{or} \qquad -(CH_2)_m-X \quad (IV)$$

in which
x in formulae III and IV in turn represents carbamoyl, alkylcarbamoyl, dialkylcarbamoyl, ureido, N'-alkylureido, N'-cycloalkylureido, N,N'dialkylureido, N' ,N' -dialkylureido or alkoxycarbonylamino and m in formula IV represents zero or an integer from 1 to 3,
b) the 1-naphthyl, 2-naphthyl, 6,7-dihydroxy-1-naphthyl, 5-hydroxy-1-naphthyl, 5,6,7,8-tetrahydro-6,7-dihydroxy-1-naphthyl, 5,6,7,8-tetrahydro-5-oxo-1-naphthyl, 9-fluorenon-4-yl or inden-4-yl radical or
c) an indolyl, methylindolyl, 2-hydroxyquinolyl, carbazolyl or 4-morpholinyl-2,1,3-thiadiazolyl radical,
$R_1$ denotes hydrogen or a straight-chain or branched $C_2$- to $C_5$-alkanoyl or aroyl radical,
$R_2$ denotes hydrogen and
$R_3$ denotes a branched $C_3$- to $C_6$-alkyl radical, an unsubstituted or hydroxyl-substituted or alkoxysubstituted straight-chain or branched phenyl-lower alkyl or indolyl-lower alkyl radical, a radical of the formula V

$$-(CH_2)_l-Y \qquad (V)$$

where, in formula V, 1 represents the integer 1 or 2 and Y represents an N'-phenylureido, N'pentamethyleneureido or N'-3''-oxapentamethyleneureido radical or an unsubstituted or substituted phenylacetylamino radical, or
$R_2$ and $R_3$, bonded to one another and together with the adjacent nitrogen atom, denote an unsubstituted or substituted 5-membered or 6-membered saturated heterocyclic radical, characterized in that an aryloxypropanolamine of the general formula VI

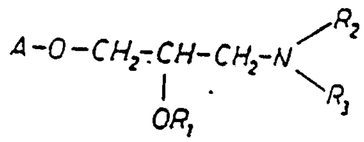

(VI)

in which
A, $R_1$, $R_2$ and $R_3$ have the meaning stated under formula I, is reacted with at least the stoichiometric amount of diphenylacetic acid in a solvent or solvent mixture which is inert to the reactants, at temperatures from room temperature to the boiling point of the solvent or of the lowest-boiling solvent component, the diphenyl acetate of the formula I which is precipitated at room temperature or preferably at low temperature is separated off from the reaction mixture obtained in the reaction and is optionally recrystallized from an organic solvent.

2. Process according to Claim 1, characterized in that N'-( 3-acetyl-4-(3-((1-dimethylethyl)-amino)-2-hydroxy-propoxy)-phenyl)-N,N-diethylurea (celiprolol), 1-((1methylethyl)-amino)-3-(1-naphthyloxy)-2-propanol (propranolol), 1-(2,3-dimethylphenoxy)-3-(1,1-dimethylethyl)-amino-2-propanol (xibenol), 1-(4-(2-methoxyphenyl)-piperazin-1-yl)-3-((3,4,5-trimethoxy)-phenoxy)-2-propanol (enciprazine), 1-(2,5-dichlorophenoxy) -3-((1,1-dimethylethyl)-amino)-2-propanol (cloranolol), l-(2-chloro-5-methylphenoxy)-3-((1,1-dimethylethyl)-amino)-2-propanol (bupranolol),1-(4-(2-methoxyethyl)-phenoxy)-3-((1-methylethyl)-amino)-2-propanol (metoprolol), 4-(2-hydroxy-3-((1-methylethyl) -amino) -propoxy)-phenyl) -acetamide (atenolol), N-(3-acetyl-4-(2-hydroxy-3-((1-methylethyl) -amino) -propoxy) -phenyl) -acetamide (diacetolol), 1((1-methylethyl)-amino)-3-(2-(2-Propenyloxy)-phenoxy)-2-propanol (oxprenolol), 1-((1,1-dimethylethyl)-amino)-3-(2-((tetrahydro-2-furanyl)-methoxy)-phenoxy)-2-propanol (bufetolol), 1-(3-methylphenoxy)-3-((2-(3,4-dimethoxyphenyl)-ethyl)-amino)-2-propanol (bevantolol), 2-(3-((1,1-dimethylethyl) -amino) -2-hydroxypropoxy) -benzonitrile (bunitrolol), N(3-acetyl-4-(2-hydroxy-3-((1-methylethyl) -amino) propoxy)-phenyl)-butyramide (acetbutolol), 1-((1,1-dimethylethyl)-amino)-3-((4-(morpholinyl) -2,1 ,3-thiadiazol-5-yl)-2-propanol (timolol), N-(2-((3-(2-cyanophenoxy)-2-hydroxypropyl)-amino)-ethyl)-2-(4-hydroxyphenyl)-acetamide(epanolol), 2-(2-hydroxy-3-((2(1H)-indol-3-yl-1,1-dimethylethyl)-amino)-propoxy)-benzonitrile (bucindolol), 1-(2,4-dichlorophenoxy)-3-(3,4-dimethoxy-beta-phenylethyl-amino)-propanol, 4-(2-hydroxy-3-((1-methyl-3-PhenylPropyl)-amino)-propoxy)-phenylacetamide, N-(2-((3-(2-cyanophenoxy)-2-hydroxypropyl)-amino)-ethyl)-N'-phenylurea, 5-(3-((1,1-dimethylethyl)-amino)-2-hydroxypropoxy)-3,4-dihydro-1(2H)-naphthalenone (bunolol) and 2-(3-((1-dimethylpropyl)-amino)-2-hydroxypropoxy)-benzonitrile (penirolol) is reacted with diphenylacetic acid.

3. Process according to claim 1 or 2, characterized in that a crude product as obtained by a synthesis customary for the preparation of aryloxypropanolamines, after separation from the reaction mixture, without a further purification step, is used as the aryloxypropanolamine of the formula VI.

4. Process according to any of Claims 1 to 3, characterized in that a crude product as obtained by reacting a 1-aryloxy-2,3-epoxypropane of the general formula VII

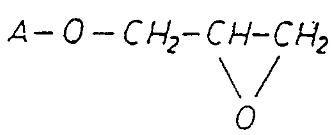

(VII)

or a compound of the general formula VIII

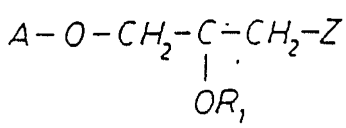

(VIII)

with the equivalent amount or a small excess, for example a two-fold to three-fold excess, of an amine of the general formula IX

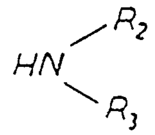

(IX)

where, in the above formulae A, $R_1$, $R_2$ and $R_3$ have the meaning stated under formula I and Z represents a leaving group which can be readily eliminated, after separation from the reaction mixture, is used as the aryloxypropanolamine of the formula VI.

**5.** Use of the salts of the general formula I prepared according to any of Claims 1 to 4 for the preparation of chemically pure aryloxypropanolamines of the general formula VI or of their pharmaceutically tolerated acid addition salts, by recovering the aryloxypropanolamine of the formula VI from the diphenyl acetate of the general formula I by the addition of bases, and, if desired, converting it with an acid into a pharmaceutically tolerated addition salt.

**6.** Use of the salts of the general formula I prepared according to any of Claims 1 to 4 for the preparation of pharmaceutically tolerated acid addition salts of aryloxypropanolamines of the general formula VI, by reacting the diphenyl acetate of the general formula I with a suitable acid to form another salt.

## Revendications

**1.** Sels d'aryloxypropanolamine avec l'acide diphénylacétique répondant à la formule générale I:

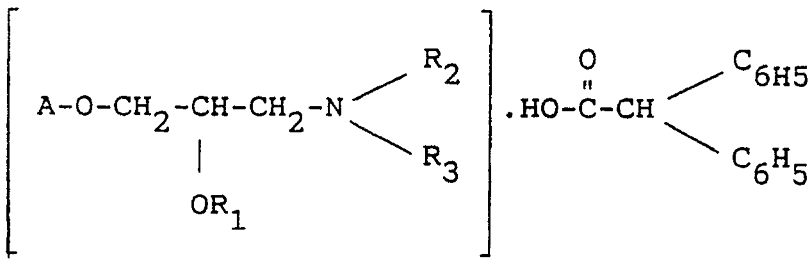

(I)

dans laquelle:
A signifie:
a) un radical phényle substitué de formule générale II:

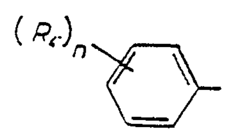

(II)

dans laquelle:
n représente un nombre entier compris entre 1 et 4; les substituants $R_4$ sont soit identiques, soit différents et, indépendamment les uns des autres, représentent un radical alkyle inférieur, alkényle inférieur en $C_2$ ou $C_3$, cycloalkyle en $C_5$ ou $C_6$, alkoxy inférieur, alkénoxy inférieur en $C_2$ ou $C_3$, cycloalkylalkoxy, tétrahydrofuranylméthoxy, alkoxy inférieur-alkyle inférieur, alkanoyle inférieur, un atome d'halogène, un groupe hydroxy, cyano, carbonamido, acyloxy, des restes de formule III ou IV:

$$-O-CH_2-X \quad (III) \quad ou \quad -(CH_2)_m-X \quad (IV)$$

où:
X, dans les formules III et IV, représente également des radicaux carbamoyle, alkylcarbamoyle, dialkylcarbamoyle, uréido, N'-alkyluréido, N'-cycloalkyluréido, N,N'-dialkyluréido, N',N'-dialkyluréido ou alkoxycarbonylamino; et m, dans la formule IV, est égal à 0 ou à un nombre entier compris entre

1 et 3.

b) le radical l-naphtyle, 2-naphtyle, 6,7-dihydroxy-l-naphtyle, 5-hydroxy-1-naphtyle, 5,6,7,8-tétrahydro-6,7-dihydroxy-1-naphtyle, 5,6,7,8-tétrahydro-5-oxo-1-naphtyle, 9-fluorénon-4-yl ou un radical indèn-4-yl, ou

c) un radical indolyle, méthylindolyle, 2-hydroxy-chinoléine, carbazolyle ou 4-morpholinyl-2,1,3-thiadiazolyle;

$R_1$ représente un atome d'hydrogène ou un radical alkanoyle ou aroyle en $C_2$ à $C_5$ à chaîne droite ou ramifiée;

$R_2$ représente un atome d'hydrogène; et

$R_3$ représente un radical alkyle en $C_3$ à $C_6$ ramifié, un radical non substitué ou substitué par un groupe hydroxy ou alkoxy, un radical phénylalkyle inférieur à chaîne droite ou ramifiée ou un radical indolylalkyle inférieur, un reste de formule V:

$$-(CH_2)_1-Y \qquad (V)$$

dans la formule V, 1 représentant un nombre entier égal à 1 ou 2; et

Y représentant un radical N'-phényluréido, N'-pentaméthylèneuréido, N'-3"-oxapentaméthylèneuréido ou un reste phénylacétylamino non substitué ou substitué, ou $R_2$ et $R_3$ sont reliés entre eux et forment ensemble, avec l'atome d'azote auquel ils sont rattachés, un radical hétérocyclique saturé à 5 ou 6 éléments non substitués ou substitués.

2. Sels selon la revendication 1, de N'-[3-acétyl-4-[3-[(1,1-diméthyléthyl)amino]-2-hydroxypropoxy]phényl]-N,Ndiéthylurée (Céliprolol), 1-[(1-méthyléthyl)amino]-3-(1-naphtyloxy)-2-propanol (Propranolol), 1-(2,3-diméthyl-phénoxy)-3-(1,1-diméthyléthyl)amino-2-propanol (Xibénol), 1-[4-(2-méthoxyphényl)pipérazin-1-yl]-3-[(3,4,5-triméthoxy)-phénoxy]-2-propanol (Enciprazin), 1-(2,5-dichlorophénoxy)-3-[(1,1-diméthyléthyl)amino]-2-propanol (Cloranolol), 1-(2-chloro-5-méthylphénoxy)-3-[(1,1-diméthyléthyl)amino]-2-propanol (Bupranolol), 1-[4-(2-méthoxyéthyl)phénoxy]-3-[(1-méthyléthyl)amino]-2-propanol (Métoprolol), 4-[2-hydroxy-3-[(1-méthyléthyl)amino]propoxy]phénylacétamide (Aténolol), N-[3-acétyl-4-[2-hydroxy-3-[-(1-méthyléthyl)amino]propoxy]-phényl]acétamide (Diacétolol), 1-[(1-méthyléthyl)amino]-3-[2-(2-propényloxy)phénoxy]-2-propanol (Oxprénolol), 1-[(1,1diméthyléthyl)amino]-3-[2-[(tétrahydro-2-furanyl)méthoxy]-phénoxy]-2-propanol (Bufétolol), 1-(3-méthylphénoxy)-3-[[2-(3,4-diméthoxyphényl)éthyl]amino]-2-propanol (Bévantolol), 2-[3-[(1,1-diméthyléthyl)amino]-2-hydroxypropoxy]benzonitrile (Bunitrolol), N-[3-acétyl-4-[2-hydroxy-3-[(1-méthyléthyl)-amino]propoxy]phényl]butyramide (Acébutolol), 1-[(1,1diméthyléthyl)-amino]-3-[[4-(morpholinyl)-2,1,3-thiadiazol-5-yl]-2-propanol (Timolol), N-[2-[[3-(2-cyanophénoxy)-2-hydr oxy-propyl]amino]éthyl]-2-(4-hydroxyphényl)acétamide (Epanolol), 2-[2-hydroxy-3-[[2-(1H)-indol-3-yl-1,1-diméthyléthyl]amino]propoxy]benzonitrile (Bucindolol), 1-(2,4-dichlorophénoxy)-3-(3,4-diméthoxy-béta-phényléthylamino)-2-propanol, 4-[2-hydroxy-3-[(1-méthyl-3-phénylpropyl)amino]propoxy]-phényl-acétamide, N-[2-[[3-(2-cyanophénoxy)-2-hydroxypropyl]-amino]éthyl]-N'-phénylurée, 5-[3-[(1,1-diméthyléthyl)-amino]-2-hydroxypropoxy]-3,4-dihydro-1-(2H)-naphtalinon (Bunolol), 2-[3-[(1,1-diméthylpropyl)amino]-2-hydroxypropoxy]-benzonitrile (Pénirolol).

3. Procédé de préparation de sels selon les revendications 1 ou 2, caractérisé en ce que l'on fait réagir une aryloxy-propanolamine répondant à la formule générale VI:

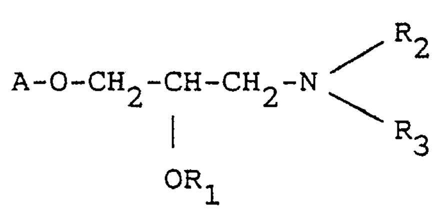

(VI)

dans laquelle:

A, $R_1$, R2 et $R_3$ présentent la signification indiquée dans la formule I,

dans un solvant ou un mélange de solvants inertes vis-à-vis des produits réagissants, à une température comprise entre la température ambiante et la température d'ébullition du solvant ou du composant du solvant dont le point d'ébullition est le plus bas, avec une quantité au moins stoechiométrique d'acide diphénylacétique, l'on sépare à la température ambiante ou de préférence à froid, du mélange

réactionnel obtenu lors de la réaction, le diphénylacétate de formule I qui s'est déposé et, le cas échéant, on le recristallise dans un solvant organique.

**4.** Procédé selon la revendication 3, caractérisé en ce que l'on emploie comme aryloxypropanolamine de formule VI un produit brut tel qu'on l'obtient par une voie de synthèse habituelle à la préparation des aryloxypropanolamines après séparation du mélange réactionnel et sans autre étape de purification.

**5.** Procédé selon la revendication 4, caractérisé en ce que l'on emploie comme aryloxypropanolamine de formule VI un produit brut tel qu'on l'obtient par réaction d'un 1-aryloxy2,3-époxypropane de formule générale VII:

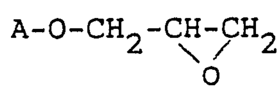

(VII)

ou d'un dérivé de formule générale VIII

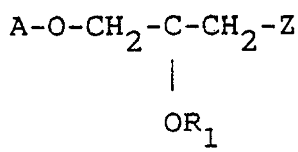

(VIII)

avec la quantité équivalente ou un faible excès, par exemple de deux à trois fois, d'une amine de formule générale IX:

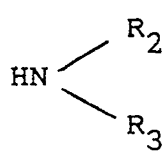

(IX)

dans laquelle, dans les formules ci-dessus:

A , $R_1$, $R_2$ et $R_3$ présentent la signification indiquée sous la formule I; et
Z représente un groupe de départ facile à cliver après la séparation du mélange réactionnel.

**6.** Utilisation des sels de formule générale I selon la revendication 1 ou 2, pour la préparation d'aryloxypropanolamines chimiquement pures de formule générale VI ou de leurs sels d'addition d'acides pharmaceutiquement compatibles, en récupérant du diphénylacétate de formule générale I l'aryloxypropanolamine de formule générale VI par addition de base et, le cas échéant, on la fait passer à l'aide d'un acide en un sel d'addition d'acide pharmaceutiquement compatible.

**7.** Utilisation selon la revendication 6, pour la préparation de sels d'addition d'acides pharmaceutiquement compatibles d'aryloxypropanolamines de formule générale VI par transformation à l'aide d'un acide approprié du diphénylacétate de formule générale I.

Revendications pour les Etats contractants suivants: AT, ES, GR

**1.** Procédé de préparation de sels d'aryloxypropanolamine avec l'acide diphénylacêtiaue à la formule générale I:

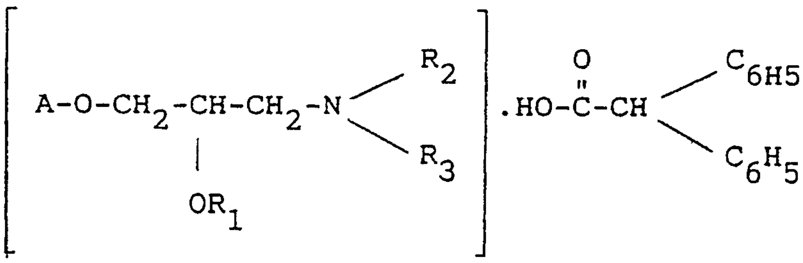

(I)

dans laquelle:

A signifie:

a) un radical phényle substitué de formule générale II:

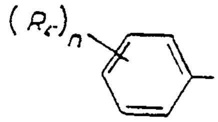

(II)

dans laquelle:

n représente un nombre entier compris entre 1 et 4; les substituants $R_4$ sont soit identiques, soit différents et, indépendamment les uns des autres, représentent un radical alkyle inférieur, alkényle inférieur en $C_2$ ou $C_3$, cycloalkyle en $C_5$ ou $C_6$, alkoxy inférieur, alkénoxy inférieur en $C_2$ ou $C_3$, cycloalkylalkoxy, tétrahydrofuranylméthoxy, alkoxy inférieur-alkyle inférieur, alkanoyle inférieur, un atome d'halogène, un groupe hydroxy, cyano, carbonamido, acyloxy, des restes de formule III ou IV:

$$-O-CH_2-X \quad (III) \quad \text{ou} \quad -(CH_2)_m-X \quad (IV)$$

où:

X, dans les formules III et IV, représente également des radicaux carbamoyle, alkylcarbamoyle, dialkylcarbamoyle, uréido, N'-alkyluréido, N'-cycloalkyluréido, N,N'-dialkyluréido, N',N'dialkyluréido ou alkoxycarbonylamino; et m, dans la formule IV, est égal à 0 ou à un nombre entier compris entre 1 et 3.

b) le radical 1-naphtyle, 2-naphtyle, 6,7-dihydroxy-1-naphtyle, 5-hydroxy-1-naphtyle, 5,6,7,8-tétrahydro-6,7-dihydroxy-1-naphtyle, 5,6,7,8-tétrahydro-5-oxo-1-naphtyle, 9-fluorénon-4-yl ou un radical indèn-4-yl, ou

c) un radical indolyle, méthylindolyle, 2-hydroxy-chinoléine, carbazolyle ou 4-morpholinyl-2,1,3-thiadiazolyle;

$R_1$ représente un atome d'hydrogène ou un radical alkanoyle ou aroyle en $C_2$ à $C_5$ à chaîne droite ou ramifiée;

$R_2$ représente un atome d'hydrogène; et

$R_3$ représente un radical alkyle en $C_3$ à $C_6$ ramifié, un radical non substitué ou substitué par un groupe hydroxy ou alkoxy, un radical phénylalkyle inférieur à chaîne droite ou ramifiée ou un radical indolylalkyle inférieur, un reste de formule V:

$$-(CH_2)_l-Y \qquad (V)$$

dans la formule V, l représentant un nombre entier égal à 1 ou 2; et Y représentant un radical N'-phényluréido, N'-pentaméthylèneuréido, N'-3"-oxapentaméthylèneuréido ou un reste phénylacétylamino non substitué ou substitué, ou $R_2$ et $R_3$ sont reliés entre eux et forment ensemble, avec l'atome d'azote auquel ils sont rattachés, un radical hétérocyclique saturé à 5 ou 6 éléments non substitués ou substitués, caractérisé en ce que l'on fait réagir une aryloxy-propanolamine répondant à la formule générale VI:

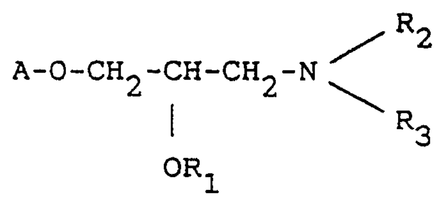

(VI)

dans laquelle:
A, $R_1$, $R_2$ et $R_3$ présentent la signification indiquée dans la formule I, dans un solvant ou un mélange de solvants inertes vis-à-vis des produits réagissants, à une température comprise entre la température ambiante et la température d'ébullition du solvant ou du composant du solvant dont le point d'ébullition est le plus bas, avec une quantité au moins stoechiométrique d'acide diphénylacétique, l'on sépare à la température ambiante ou de préférence à froid, du mélange réactionnel obtenu lors de la réaction, le diphénylacétate de formule I qui s'est déposé et, le cas échéant, on le recristallise dans un solvant organique.

**2.** Procedé selon la revendication 1, caractérisé en ce que l'on fait réagir N'-[3-acetyl-4-[3-[(1,1-diméthyléthyl)-amino]-2-hydroxypropoxy]phényl]-N,N-diéthylurée (Céliprolol), 1-[(1-méthyléthyl)amino]-3-(1-naphtyloxy)-2-propanol (Propanolol), 1-(2,3-diméthylphénoxy)-3-(1,1-diméthyléthyl)-amino-2-propanol (Xibénol), 1-[4-(2-méthoxyphényl)pipérazin-l-yl]-3-[(3,4,5-triméthoxy)-phénoxy]-2-propanol (Enciprazin), 1-(2,5-dichlorophénoxy)-3-[(1,1-diméthyléthyl)amino]-2-propanol (Cloranolol), 1-(2-chloro-5-méthylphénoxy)-3-[(1,1-diméthyléthyl)amino]-2-propanol (Bupranolol), 1-[4-(2-méthoxy-éthyl)phénoxy]3-[(1-méthyléthyl)amino]-2-propanol (Métoprolol), 4- [2-hydroxy-3-[(1-méthyléthyl)amino propoxy]phénylacetamide (Aténolol), N-[3-acétyl-4-[2-hydroxy-3-[((1-méthyléthyl)amino]propoxy]-phényl]acétamide (Diacétolol), 1-[(1-méthyléthyl)amino]-3-[2-(2-propényloxy)phénoxy]-2-propanol (Oxprénolol), 1-[(1,1-diméthyléthyl)-amino]-3-[2-[(tétrahydro-2-furanyl)méthoxy]-phénoxy]-2-propanol (Bufétolol), 1-(3-méthylphénoxy)-3-[[2-(3,4-diméthoxyphényl)éthyl]amino]-2-propanol (Bévantolol), 2-[3-[(1,1-diméthyléthyl)amino]-2-hydroxypropoxy]benzonitrile (Bunitrolol), N-[3-acétyl-4-[2-hydroxy-3-[(1-méthyléthyl)-amino]propoxy]-phényl]butyramide (Acébutolol), 1-[(1,1-diméthyléthyl)amino]-3-[[4-(morpholinyl)-2,1,3-thiadiazol-5-yl]-2-propanol (Timolol), N-[2-[[3-(2-cyanophénoxy)-2-hydroxy-propyl]amino]éthyl]-2-(4-hydroxyphényl)-acétamide (Epanolol), 2-[2-hydroxy-3-[[2-(1H)-indol-3-yl-1,1-diméthyléthyl]amino]-propoxy]benzonitrile (Bucindolol), 1-(2,4-dichlorophénoxy)-3-(3,4-diméthoxy-béta-phényléthylamino)-2-propanol, 4-[2-hydroxy-3-[(1-méthyl-3-phénylpropyl)amino]propoxy]-phényl-acétamide, N-[2-[[3-(2-cyanophénoxy)-2-hydroxypropyl]-amino]éthyl]-N'-phénylurée, 5-[3-[(1,1-dimétnylethyl)amino]-2-hyroxypropoxy]-3,4-dihydrol-1-(2H)-naphtalinon (Bunolol), 2-[3-[(1,1-diméthylpropyl)amino]-2-hydroxypropoxy]-benzonitrile (Pénirolol) avec l'acide diphényl-acetique.

**3.** Procédé selon les revendications 1 ou 2, caractérisé en ce que l'on emploie comme aryloxypropanolamine de formule VI un produit brut tel qu'on l'obtient par une voie de synthèse habituelle à la préparation des aryloxypropanolamines après séparation du mélange réactionnel et sans autre étape de purification.

**4.** Procédé selon l'une quelconque des revendications 1 à 3, caractérisé en ce que l'on emploie comme aryloxypropanolamine de formule VI un produit brut tel qu'on l'obtient par réaction d'un l-aryloxy-2,3-époxypropane de formule générale VII:

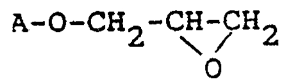

(VII)

ou d'un dérivé de formule générale VIII

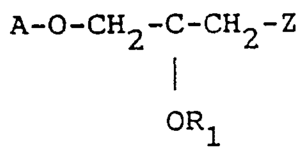

(VIII)

avec la quantité équivalente ou un faible excès, par exemple de deux à trois fois, d'une amine de formule générale IX:

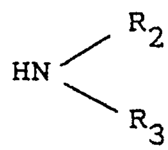

(IX)

dans laquelle, dans les formules ci-dessus:

A, $R_1$, $R_2$ et $R_3$ présentent la signification indiquée sous la formule I; et

Z représente un groupe de départ facile à cliver après la séparation du mélange réactionnel.

**5.** Utilisation des sels de formule générale I produits selon l'une quelconque des revendications 1 à 4, pour la préparation d'aryloxypropanolamines chimiquement pures de formule générale VI ou de leurs sels d'addition d'acides pharmaceutiquement compatibles, en récupérant du diphénylacétate de formule générale I l'aryloxypropanolamine de formule générale VI par addition de base et, le cas échéant, on la fait passer à l'aide d'un acide en un sel d'addition d'acide pharmaceutiquement compatible.

**6.** Utilisation des sels de formule générale I produits selon l'une quelconque des revendications 1 à 4, pour la préparation de sels d'addition d'acides pharmaceutiquement compatibles d'aryloxypropanolamines de formule générale VI par transformation à l'aide d'un acide approprié du diphénylacétate de formule générale I.